# EUROPEAN PATENT APPLICATION

(11) **EP 2 011 783 A1**
(43) Date of publication of application: **07.01.2009**
(21) Application number: 07111894.7
(22) Date of filing: 06.07.2007
(51) Int. Cl.: C07C 271/28, C07D 209/08, C07F 7/08

(54) **Process for the preparation of an indole derivative**

(71) Applicant: F. Hoffmann-La Roche AG, 4070 Basel (CH)
(72) Inventor: Stephan Bachmann, 4123 Allschwil (CH); Goesta Rimmler, 79189 Bad Krozingen (DE); Philippe Pflieger, 68130 Schwoben (FR); Michelangelo Scalone, 4127 Birsfelden (CH)
(74) Representative: Heiroth, Ulrike Hildegard

(57) **Abstract**

A process for the preparation of indole derivatives of formula (I): 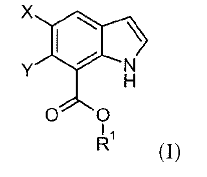 which are useful as intermediates in the preparation of i.a. pharmaceutically active compounds.

## Description

The present invention relates to a process for the preparation of indole derivatives which are useful as intermediates in the preparation ofpharmaceutically active compounds.

In a first aspect, the present invention provides a process for the preparation of an indole derivative of formula (I): 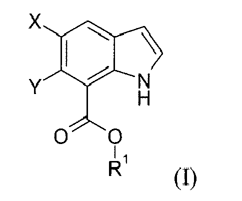 wherein
X is halogen, (C₁-C₆)alkoxy (preferably (C₁-C₃)alkoxy) or (C₁-C₆)alkyl (preferably (C₁-C₃)alkyl);
Y is halogen or (C₁-C₆)alkyl (preferably (C₁-C₃)alkyl);
R¹ is (C₁-C₆)alkyl, (C₃-C₆)cycloalkyl, halo(C₁-C₆)alkyl, perhalo(C₁-C₆)alkyl, (C₁-C₆)alkenyl, (C₁-C₆)alkynyl, arylalkyl or optionally substituted aryl
which comprises the cyclization of formula (II) compound: 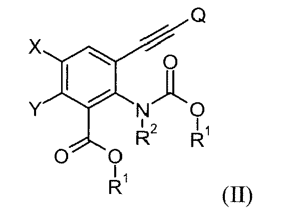 wherein X, Y and R¹ are as defined above, and
R² is halo(C₁-C₆)alkyl, perhalo(C₁-C₆)alkyl, -CO₂R, COR, or-SO₂R' wherein R and R' are (C₁-C₆)alkyl, phenyl(C₁-C₃)alkyl (preferably benzyl), (C₁-C₃)alkylphenyl or phenyl;
Q is -Si(R³)(R⁴)(R⁵) or -C(OH)(R⁴)(R⁵) wherein R³, R⁴ and R⁵ may be the same or different and are independently selected from (C₁-C₆)alkyl or phenyl, more preferably R³, R⁴ and R⁵ are (C₁-C₆)alkyl, most preferably Me;
with a cyclization reagent such as alcoholate, alkalimetalhydroxyl, tetra(C₁-C₁₀)alkyl ammonium salts, alkali metal carbonates such as K₂CO₃ or Cs₂CO₃ or a mixture thereof.

Preferably Q is -Si(R³)(R⁴)(R⁵) wherein R³, R⁴ and R⁵ are as defined previously, most preferably trimethylsilane.

Preferably R² is -CO₂R, most preferably -CO₂Me.

Preferably R¹ is (C₁-C₆)alkyl, (C₃-C₆)cycloalkyl or arylalkyl, more preferably (C₁-C₃)alkyl, most preferably methyl.

More preferably X and Y are independently halogen or (C₁-C₃)alkyl, most preferred halogen. The cyclization may be followed by the hydrolysis of the ester by using well known saponifaction methods.

The cyclization may be performed in the presence of a solvent, such as an alcohol (e.g. methanol or ethanol), an ether-like solvent (e.g. tetrahydrofuran, diisopropyl ether, t-butylmethyl ether, dibutyl ether, dimethyl acetal or dioxane), ester-like solvent (e.g. ethyl acetate), aromatic solvent (e.g. toluene or t-butyl-benzene), an aliphatic hydrocarbon solvent (e.g. hexanes, heptanes or pentane), a saturated alicyclic hydrocarbon solvent (e.g. cyclohexane or cyclopentane), acetonitrile, an aprotic polar solvents (e.g.dimethylformamide), or Dimethyl Sulfoxide, preferably in the presence of methanol, ethanol, iso-propanol, tert-butanol, tetrahydrofuran, toluene or dimethylformamide, more preferred in the presence of ethanol.

The cyclization reagent may be present in an amount from 0.1-5 molar equivalents, preferably from 0.2-1.2 molar equivalents, more preferably 0.2 molar equivalents.

The cyclization reaction may be performed at a temperature from 20-120°C, e.g. from ambient temperature to 120°C, e.g. at 80°C.

The compound of formula (I) may be used as an intermediate in the synthesis of valuable pharmaceutical compounds, such as the ones described in WO2006/013048.

Unless otherwise stated, the following terms used alone or as part of another group in the specification and claims have the meanings given below:
"aryl" refers to a monovalent monocyclic or bicyclic aromatic hydrocarbon moiety which is optionally substituted with one or more, preferably one, substituents each of which is preferably selected from the group consisting of (C₁-C₆)alkyl, (C₁-C₆)alkoxy, nitro, halo, -C(O)O(C₁-C₆)alkyl. A particular preferred aryl substituent is halo. More preferably the term aryl means phenyl, 1-naphtyl, 2-naphtyl, each of which can be substituted. Most preferably aryl is an optionally substituted phenyl.
"(C₁-C₆)alkyl" refers to a branched or straight hydrocarbon chain, such as methyl, ethyl, n-propyl, isopropyl, n-butyl, isobutyl, sec-butyl and t-butyl, pentyl, hexyl.
"(C₁-C₆)alkenyl" means straight or branched carbon chains having one or more double bonds in the chain, such as vinyl, 3-Butenyl, allyl, isopropenyl 2,2-dimethylene, propylene, 2-methylpropylene, butylene, pentylene. The more preferred alkenyl is vinyl, 3-Butenyl, allyl or isopropenyl.
"Alkynyl" means straight or branched carbon chains having one or more triple bonds in the chain, such as propargyl, 2-Butynl-yl, 3-Butyn-1-yl.
"Arylalkyl" refers to a moiety of the formula Ar-R^{x}- where Ar is optionally substituted aryl and R^{x} is alkyl as defined herein. Preferably arylakyl is optionally substituted benzyl.
"(C₃-C₆)cycloalkyl" refers to a single saturated carbocyclic ring, such as cyclopropyl, cyclobutyl, cyclopentyl and cyclohexyl each of which are optionally substituted with one or more, preferably one, selected from the group consisting of (C₁-C₆)alkyl, (C₁-C₆)alkoxy, halo.
"(C₁-C₆)alkoxy" is understood as being an -O-(C₁-C₆)alkyl wherein (C₁-C₆)alkyl is as above defined, such as methoxy, ethoxy, isopropoxy.
"halo(C₁-C₆)alkyl" is understood as being an (C₁-C₆)alkyl chain as above defined substituted by one or more, same or different halogen atoms, such as 2-Br or 2-Cl-ethyl, 1-Cl-ethyl, 4-Cl-butyl, 2,2,2-trichloror-1,1-dimethylethyl.

The term "perhalo" means all hydrogens have been replaced with halogen atoms.

The term "Alcoholate" refers to alkalimetal conjugate of (C₁-C₈)alcohol, such as NaOMe, NaOEt, NaOiPr, NaOnPr, NaOtBu, NaOnBu, NaOnHexyl, sodium tert-amylate, NaDMO (sodium 3,7-dimetheyl-3-octylate) and their corresponding potassium salt analogues (such as KOtBu). A preferred alcoholate is NaOMe, NaOEt, NaOiPr or KOtBu. Of these, sodium ethoxide is the most preferred.

"Alkalimetalhydroxyl" refers to alkalimetal-OH salt, such as KOH, NaOH.

The term "alkalimetal-amine", refers to a secondary amine substituted by an alkali metal as defined herein. The "alkalimetal-amine" is either prepared *in situ* or *ex situ* prior to use, following synthetic routes well known by the person skilled in the art or it is available commercially. The most preferred alkali metal to be used is lithium. Exemplary alkalimetal-amine includes lithium adducts of dicyclohexyl amine, diisopropyl amine, tetramethyl piperidine or hexamethyl disilazane. The most preferred alkalimetal-lithium is lithium dicyclohexyl amine.

The term "alkali metal" includes lithium, sodium and potassium. Preferable alkali metal is lithium or sodium.

The term "halo" or "halogen" means fluorine, chlorine, bromine or iodine, preferably fluorine or chlorine.

The term "acid quenching reagent" refers to an acid that covalently combines with a reactant (e.g. to form a salt) to make it less reactive, such as acetic acid or citric acid.

"Secondary amine" refers to an amine of formula (a) 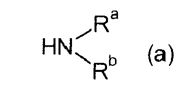 where R^{a} and R^{b} may be the same or different and are independently selected from (C₁-C₆)alkyl, (C₃-C₆)cycloalkyl or -Si(C₁-C₆)alkyl, or R^{a} and R^{b} taken together with the nitrogen atom to which they are attached, form a (C₄-C₈)heterocycloalkane optionally containing an additional heteroatom selected from O or N. Representative examples include, but are not limited to, piperidine, 4-methyl-piperidine, piperazine, pyrrolidine, morpholine, dimethylamine, diethylamine, diisopropylamine, dicyclohexylamine, ethylmethylamine, ethylpropylamine, methylpropylamine and hexamethyl disilazide. Preferably, the secondary amine is chosen from diethylamine, diisopropylamine, dicyclohexylamine, ethylmethylamine, ethylpropylamine, methylpropylamine and morpholine. The most preferred secondary amine is dicyclohexyl amine.

"(C₄-C₈)heterocycloalkane" refers to a saturated non-aromatic cyclic compound of 4 to 8 ring atoms in which one or two ring atoms are heteroatoms selected from N or O, and the heterocycloalkane may be optionally substituted with one or more (C₁-C₃)alkyl, preferably one (C₁-C₃)alkyl.

"Tetra(C₁-C₁₀)alkyl ammonium salts" refers to a quaternary ammonium ([alkyl₄N]⁺) wherein alkyl is a straight hydrocarbon radical chain containing 1 to 10 carbon atoms, preferably 2 to 6 carbon atoms, most preferably 3 carbon atoms, and wherein the counter ion is selected from hydroxide, halogen, hydrogensulfate, borohydride, cyanoborohydride, periodate, dihydrogentrifluoride, difluorotriphenyl stannate, trifluoromethanesulfonate, hexafluorophosphate, tetrafluoroborate, nitrate, perchlorate, phosphate. The counter ion is preferably a halogen, most preferably fluoride ion. Representative examples include, but are not limited to, a tetrabutylammonium hydroxide, tetra-n-butylammonium tribromide, tetrabutylammonium iodide, tetrabutylammonium hydrogen sulphate, tetrabutylammonium chloride, tetrabutylammonium bromide, tetrabutylammonium fluoride, tetrabutylammonium borohydride, tetrabutylammonium fluoride trihydrate, tetrabutylammonium cyanoborohydride, tetrabutylammonium periodate, tetrabutylammonium dihydrogentrifluoride, tetrabutylammonium difluorotriphenyl stannate, tetra-n-butylammonium trifluoromethanesulfonate, tetra-n-butylammonium hexafluorophosphate, tetrabutylammonium, tetrafluoroborate, tetrabutylammonium nitrate, tetrabutylammonium perchlorate, tetrabutylammonium phosphate, tetra-n-butylammonium trifluoromethanesulfonate, tetraethylammonium chloride hydrate, tetraethylammonium p-toluenesulfonate, tetraethylammonium bromide, tetraethylammonium hydroxide, tetraethylammonium acetate tetrahydrate, tetraethylammonium fluoride hydrate, tetraethylammonium iodide, tetraethylammonium tetrafluoroborate, tetraethylammonium hydrogensulfate, tetraethylammonium nitrate, tetraethylammonium perchlorate, tetrapropylammonium bromide, tetrapropylammonium iodide, tetrapropylammonium chloride, tetrapropylammonium hydroxide, tetrapropylammonium perchlorate, tetrapentylammonium bromide, tetrahexylammonium benzoate, tetrahexylammonium bromide, tetrahexylammonium chloride, tetrahexylammonium iodide, tetraheptylammonium bromide, tetraheptylammonium chloride and tetraheptylammonium iodide.

In another embodiment, the present invention provides a process for the preparation of the compound of formula (II): 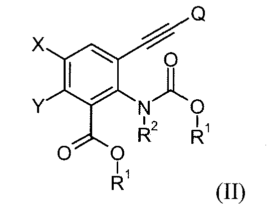 which comprises the carboxylation of the compound of formula (III): 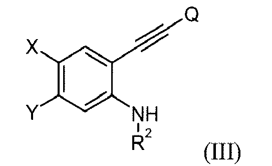 wherein X, Y, Q, R¹ and R² are as defined above, with a compound of formula Z-C(O)O- R¹ wherein Z is a halogen (preferably chlorine) and R¹ is as defined previously, in the presence of alkalimetal-amine, optionally followed by the addition of an acid quenching reagent. Preferably the carboxylation of the compound of formula (III) is followed by the addition of an acid quenching reagent.

The compound of formula (III) is synthesised according to procedures analogous to those described by Kenichi Sonogashira, in J. Organomet. Chem. 2002, 653, 46. For example to a mixture of Palladium catalyst, such as PdCl₂(PPh₃)₂, Pd(PPh₃)₄, PdCl₂(dppf) and Pd/C and/or of copper catalysts such as CuI and CuBr or a mixture of Na₂PdCl₄, CuI and R₃PHBF₄ (preferably with a ratio 4:3:8, R = tBu or Adamantyl) or a Perovskite catalyst (e.g. LaFe_{0.57}M_{0.38}Pd_{0.05}O₃, M = Co, Cu) in the presence of solvent such as triethylamine, diethylamine, ammonia (aq.), alcohol (e.g. methanol or ethanol), an ether-like solvent (e.g. tetrahydrofuran, diisopropyl ether, t-butylmethyl ether, dibutyl ether, dimethyl acetal or dioxane), an aprotic polar solvents (e.g.dimethylformamide), and ester-like solvent (e.g. ethyl acetate), a compound of formula (IV) is added followed by the addition of acetylene of formula (B), as shown in scheme 1 below. Preferably the acetylene coupling is performed with PdCh(PPh₃)₂ and CuI as catalyst in the presence of triethylamine. In scheme 1, X, Y, R² and Q are as defined above. 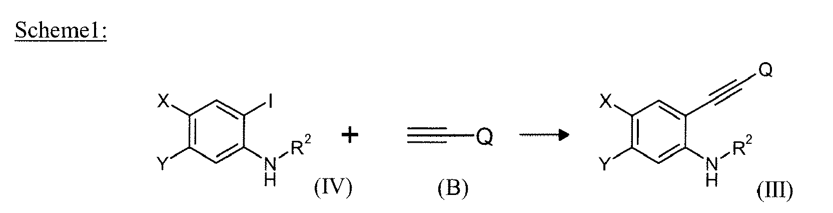 In a further embodiment the present invention provides a process for the preparation of the compound of formula (IV), which comprises iodination of the compound of formula (V): 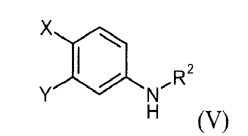 wherein X, Y and R² are as defined previously, with an iodination source (e.g.iodine/NaIO₄, iodine/H₅IO₆, N-iodo succinimide, N-iodo phthalimide, 1,3-diiodo-5,5-dimethylhydantoin, ICl and HIO₃ or a mixture thereof). Preferably the iodination source is selected from iodine/NaIO₄, iodine/H₅IO₆, N-iodo succinimide, N-iodo phthalimide and 1,3-diiodo-5,5-dimethylhydantoin, Most preferably the iodination source is iodine/NaIO₄.

Preferably the iodination is carried out in the presence of acid such as sulfuric acid, methane sulfonic acid, triflic acid, hydrochloric acid or nitric acid, more preferably sulfuric acid, methane sulfonic acid or triflic acid, most preferably sulfuric acid. The acid may be present in an amount from 0.1-100 equivalents, more preferably from 0.2-1 equivalents, most preferably 0.5 molar equivalents.

For the iodination reaction, depending on the particular iodine source the amount thereof in the reaction mixture may be, e.g.:
For iodine/NaIO₄: iodine in the range from 0.4-1.5 eq. and NaIO₄ in the range from 0.2-1.5 eq.; more preferably iodine from 0.4-1.0 eq. and NaIO₄ from 0.2-0.6 eq.; most preferably iodine 0.5 eq. and NaIO₄ 0.3 eq.;
For iodine/H₅IO₆: iodine in the range from 0.3-1.5 eq. and H₅IO₆ in the range from 0.2-1.5 eq., most preferably for iodine from 0.3-1 eq., for H₅IO₆ from 0.2-0.6 eq.;
For N-iodo succinimide: In the range from 1-5 eq., most preferably from 1-1.5 eq.;
For 1,3-diiodo-5,5-dimethythydantoin: In the range from 0.5-2 eq., most preferably from 0.5-0.8 eq.

The iodination may be carried out in the presence of organic solvent such as acetonitrile, chlorinated hydrocarbons (e.g. chloroform, dichloromethane), an ether-like solvent (e.g. tetrahydrofuran, diisopropyl ether, t-butylmethyl ether, dibutyl ether, dimethyl acetal or dioxane), such as an alcohol (e.g. methanol or ethanol), an aprotic polar solvents (e.g. dimethylformamide), ester-like solvent (e.g. ethyl acetate), and acetic acid. Preferably the solvent during the iodination is acetonitrile, ethyl acetate, methanol, dichloromethane or tetrahydrofuran, most preferably acetonitrile.

The protection of the free amino group of compound (VI) is carried out according to methods well known to those skilled in the art (see for instance P. J. Kocienski, Protecting Groups, Thieme Verlag Stuttgart, 1994 [Chapter 6] or T. W. Greene, P. G. M. Wuts, Protective Groups in Organic Synthesis, John Wiley & Sons New York, 3rd ed., 1999 (Chapter 7)). For example to a solution of compound of formula (VI) in a solvent such as, halogenated solvent, ether-like solvent or/and water, may be added firstly a base such as carbonic acid salts (e.g. sodium bicarbonate, potassium carbonate), tertiary amines (such as triethylamine, tributylamine, diethylmethylamine, dimethyl-ethylamine and methylethylbutylamine, triethylamine, diisopropylethylamine, pyridine), secondary amines as defined previously, a primary amine (e.g. benzylamine, 1-phenyl-benzylamine), diamines (e.g. ethylene diamine, tetramethylethylene diamine), salts of carboxylic acids (e.g. NaOAc) or a mixture thereof, followed by the addition of a compound of formula (VIIa) or (VIIb) as shown in scheme 2 below. Preferably the base is sodium bicarbonate. The base may be present in an amount of from 0.1-100 equivalents, preferably 1.05 molar equivalents. In scheme 2, Z is a halogen, most preferably Cl, and X, Y and

R² are as defined above.

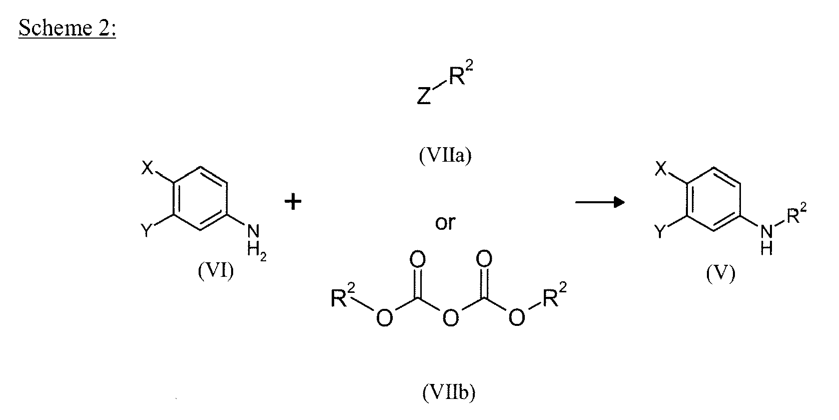

In a further embodiment the present invention provides a process for the preparation of the compound of formula (I), which comprises the carboxylation followed by the cyclization steps, as previously disclosed and following scheme 3, wherein X, Y, Q, R¹ and R² are as defined above.

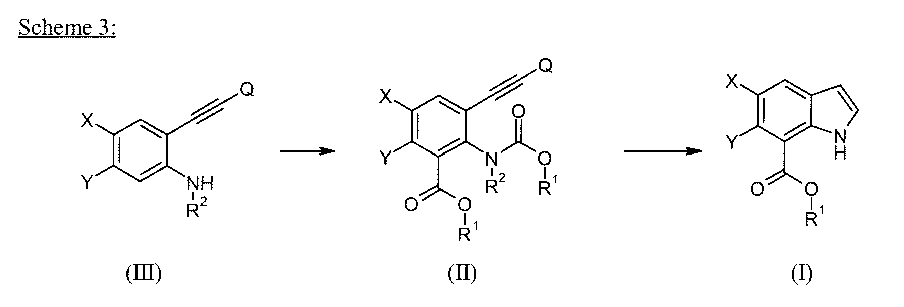

In another embodiment the present invention provides a process for the preparation of the compound of formula (I), which comprises the following steps:
1) the Sonogashira reaction of compound of formula (IV), as previously described;
2) the carboxylation of compound of formula (III), as previously described;
3) the cyclization of compound of formula (II), as previously described;
wherein X, Y, Q, R¹ and R² are as defined above and the process is carried out according to scheme 4.

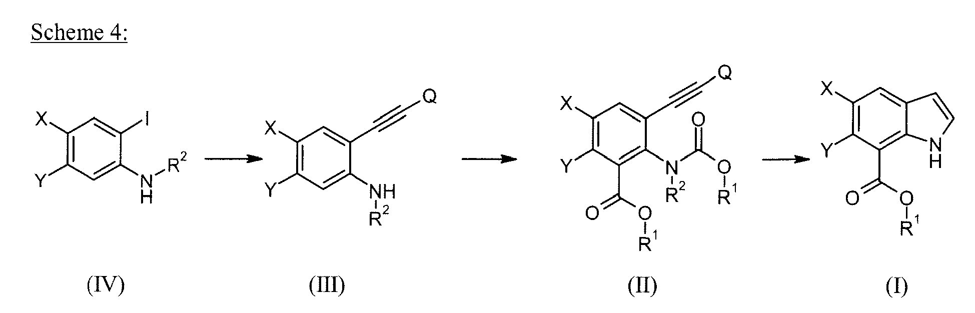

In another embodiment, the present invention provides a process for the preparation of the compound of formula (I), which comprises the following steps:
1) the iodination of compound of formula (V), as previously described;
2) the Sonogashira reaction of compound of formula (N), as previously described;
3) the carboxylation of compound of formula (III), as previously described;
4) the cyclization of compound of formula (II), as previously described;
wherein X, Y, Q, R¹ and R² are as defined above and the process follows scheme 5. 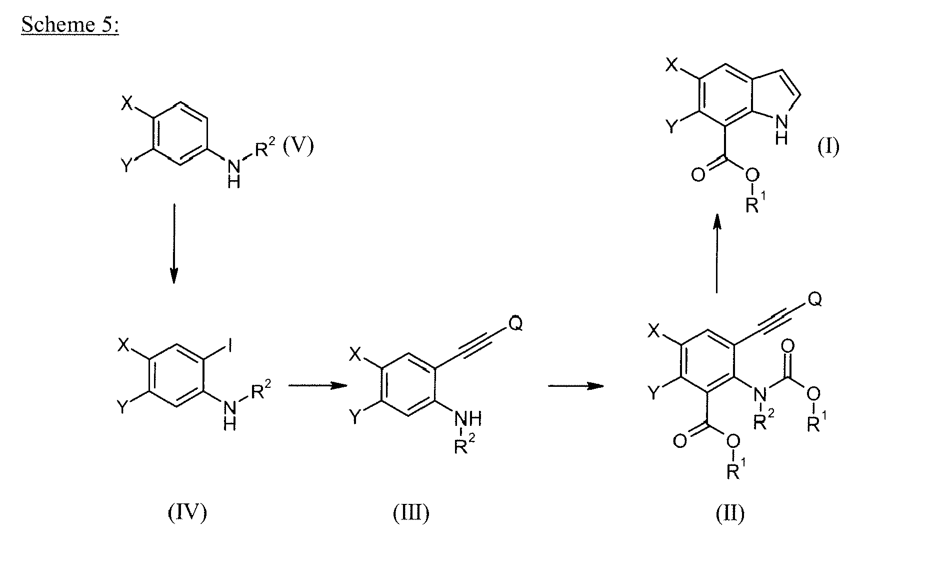 In a further embodiment, the present invention provides a process for the preparation of an indole derivative of formula (I): 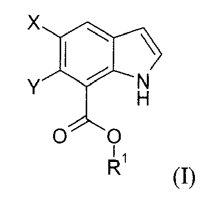 which comprises the cyclization of formula (II') compound: 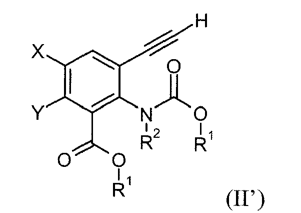 wherein X, Y, R¹ and R² are as defined above, with a cyclization reagent as defined previously.

The compound of formula (II') is synthesized according to procedures well known to those skilled in the art (see for instance J. Wettergren et. al Tetrahedron Lett. 2003, 44, 7611 or M. Ansorge et. al J. Organomet. Chem. 1999, 585, 174). For example to a solution of compound of formula (II) wherein Q is a silyl protecting group, in a solvent such as alcohols (e.g. methanol), ether-like solvent (e.g. THF) or/and water, is added for example K₂CO₃ or TBAF.

In yet another embodiment of the present invention, an intermediate having formula (II") is provided, 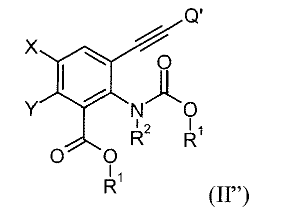 wherein R¹ and R² are as defined above, and
X is halogen, (C₁-C₆)alkoxy or (C₁-C₆)alkyl;
Y is halogen or (C₁-C₆)alkyl;
Q' is H, -Si(R³)(R⁴)(R⁵) or -C(OH)(R⁴)(R⁵) wherein R³, R⁴ and R⁵ are as defined previously. Preferably Q' is Q as defined above.

Preferably a compound of formula (II') wherein X and Y are independently halogen or (C₁-C₃)alkyl; most preferably fluorine or chlorine.

The starting materials and reagents, which do not have their synthetic route explicitly disclosed herein, are generally available from commercial sources or are readily prepared using methods well known to the person skilled in the art. For instance, DIH was synthesized according to the literature procedure O.O. Orazi, R.A. Rorral, H.E. Bertorello, J. Org. Chem. 1965, 30, 1101.

The following examples are provided for the purpose of further illustration and are not intended to limit the scope of the claimed invention.

The following abbreviations and definitions are used: AcOEt (ethyl acetate); AcOH (acetic acid); aq. (aqueous); br (broad); BuLi (butyllithium); calc. (calculated); CDCl₃ (deuterated chloroform); DCM (dichloromethane); DEA (diethylamine); DIH (1,3-diiodo-5,5-dimethyl hydantoin); DMF (dimethylformamide); EA (elemental analysis); eq. (equivalent); Et (ethyl); g (gram); GC (gas chromatography); h (hour); HCl (hydrochloric acid); iPr (isopropyl); M (molar); m (multiplet); m/e (mass to charge ratio); Me (methyl); MeCN (acetonitrile); MeOH (methanol); ml (milliliter); m.p. (melting point); MS (mass spectroscopy); NaHCO₃ (sodium bicarbonate); NaOAc (sodium acetate); NaOH (sodium hydroxide); NaOEt (sodium ethylate); NaOMe (sodium methylate); NaOtBu (sodium tert-butylate); Na₂SO₄ (sodium sulfate); Na₂SO₃ (sodium sulfite); NEt₃ (triethylamine); NIS (*N*-Iodosuccinimide); NMR (nuclear magnetic resonance); Pd/C (palladium on carbon); PdCl₂(dppf) ([1,1'-Bis(diphenylphosphino)ferrocene] dichloropalladium); PdCl₂(PPh₃)₂ (bis(triphenylphosphine)palladiumdichloride); Ph (phenyl); RT (room temperature); s (singlet); S/C (substrate/catalyst ratio); t (triplet); TBAF (tetrabutylammoniumfluoride); TBME (t-butyl methyl ether); tBu( tert-Butyl); TfOH (triflic acid); THF (tetrahydrofuran);

### Example 1.1: Synthesis of (4-Chloro-3-fluoro-phenyl)-carbamic acid methyl ester

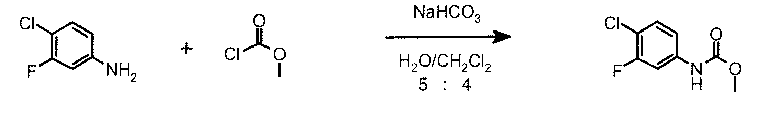

To a stirred dark violet solution of 50.0 g (344 mmol) 4-chloro-3-fluoro aniline in 400 ml DCM was added rapidly a solution of 30.3 g (361 mmol, 1.05 eq.) NaHCO₃ in 500 ml of water. To the resulting biphasic mixture was added under vigorous stirring 29.7 ml (378 mmol, 1.1 eq.) methyl chloroformate over a period of 30 min. The dark violet reaction mixture was stirred overnight at

RT and then transferred to a 2 1 separation funnel.

The organic phase was separated and washed twice with a total amount of 250 ml water, and the combined aqueous phases were washed twice with a total amount of 200 ml DCM. The combined organic phases were dried over 5 g Na₂SO₄ and filtered with suction on a funnel with a fritted disk. The cake was washed with five 15 ml portions, in total with 75 ml DCM. The DCM was partially removed under reduced pressure to about 1/3 of its original volume and then 375 ml heptane was added dropwise under stirring within 30 min, whereby the product precipitated.

From this suspension, DCM was completely removed under reduced pressure. Finally, the suspension was stirred in an ice bath for 1h, the crystals were filtered off with suction on a funnel with a fritted disk and washed with three 25 ml portions, in total with 75 ml ice cold heptane. The wet red-violet crystals were dried at 50°C *in vacuo* to yield 69.3 g (98.9% of theory) of the title compound as red-violet crystals (m.p. = 112-113°C).
¹H-NMR data (CDCl₃, 400 MHz): δ 7.44 (dd, 1H), 7.28 (dd, 1H,), 6.98 (dd, 1H), 6.64 (br s, 1H), 3.79 (s, 3H).
MS m/e (%): 203 (M⁺, 100).
EA for C₈H₇ClFNO₂ calc.: C 47.19, H 3.47, N 6.88. Found: C 46.94, H 3.22, N 6.91.
In a similar manner the reactions in Table 1.1 were performed:

**Table 1.1:**

| CO₂R | Solvent | Scale (g) | Eq. ClCO₂R | Yield (%)*^{a)}* | MS m/e (%) |
|---|---|---|---|---|---|
| Me | TBME | 10 | 1.1 | 93 | 203 (M⁺, 100) |
| Et | DCM | 10 | 1.5 | 94 | 217 (M⁺, 100) |
| *i*-Pr | DCM | 15 | 1.5 | 84.1 | 231 (M⁺, 100) |
| *t*-Bu | DCM | 5 | --*^{b)}* | 78.8 *^{c)}* | 246 ([M+H]⁺, 12) |
| CH₂Ph | DCM | 5 | 1.5 | 91.1 | 279 (M⁺, 10) |
| (-)-Menthyl | DCM | 1.5 | 1.1 | 94.4 | 327 (M⁺, 28) |

| | | | | | |
|---|---|---|---|---|---|
| *^{a)}* Typically a reaction time of 3-4 h was accomplished. *^{b)}* di-*tert*-butyl dicarbonate was used. *^{c)}* | | | | | |

The reaction mixture was stirred at ambient temperature overnight (18h) and additionally for 48h at 45°C.

### Example 1.2: Synthesis of (3-Chloro-4-fluoro-phenyl)-carbamic acid methyl ester

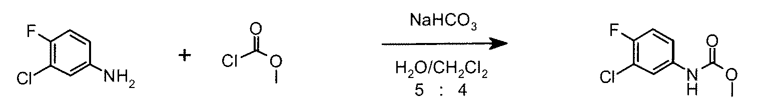

To a stirred brown solution of 25.0 g (171.8 mmol) 3-chloro-4-fluoro aniline in 200 ml DCM was added rapidly a solution of 15.2 g (180.4 mmol, 1.05 eq.) NaHCO₃ in 250 ml of water. To the resulting biphasic mixture was added under vigorous stirring 20.2 ml (257.7 mmol, 1.5 eq.) methyl chloroformate over a period of 30 min. The brown reaction mixture was stirred at RT for 2.5 h and then transferred to a 2 1 separation funnel.

The organic phase was separated and washed twice with a total amount of 125 ml water, and the combined aqueous phases were washed twice with a total amount of 100 ml DCM. The combined organic phases were dried over 2.5 g Na₂SO₄ and filtered with suction on a funnel with a fritted disk. The cake was washed with five 25 ml portions, in total with 65 ml DCM. The DCM was partially removed under reduced pressure to about 1/3 of its original volume and then 200 ml heptane was added dropwise under stirring within 30 min, whereby the product precipitated. From this suspension, DCM was completely removed under reduced pressure. Finally, the suspension was stirred in an ice bath for 1h, the crystals were filtered off with suction on a funnel with a fritted disk and washed with three 15 ml portions, in total with 45 ml ice cold heptane. The wet grey crystals were *dried in vacuo* at 50°C to yield 29.9 g (85.4% of theory) of the title compound as grey crystals (m.p. = 90.2-91.9°C).
¹H-NMR data (CDCl₃, 400 MHz): δ 7.54 (m, 1H), 7.18 (m, 1H), 7.07 (dd, 1H), 6.56 (br s, 1H), 3.78 (s, 3H).
MS m/e (%): 203 (M⁺, 100).
EA for C₈H₇ClFNO₂ calc.: C 47.19, H 3.47, N 6.88. Found: C 47.08, H 3.19, N 6.90.

### Example 1.3: Sulfonylation: Synthesis of N-(4-Chloro-3-fluoro-phenyl)-methanesulfon-amide

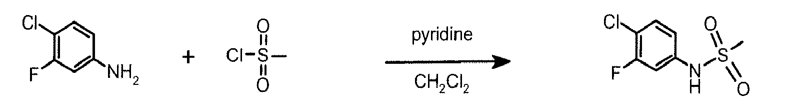

To a stirred solution of 20 g (137.4 mmol) 4-Chloro-3-fluoro aniline in 150 ml DCM was added 22.1 ml (274.8 mmol, 2 eq.) pyridine in one portion. The resulting solution was cooled to 2-4°C in an ice bath and a solution of 11.9 ml (151.2 mmol, 1.1 eq.) methane sulfochloride in 50 ml DCM was added dropwise within 1 h, whereas the temperature was kept between 2 and 4°C. A color change from brownish over yellow and orange towards red was observed.

After additional stirring for 3 h 200 ml water was added to the reaction mixture, the organic phase was washed with 150 ml water and the aq. phase was washed with a total amount of 150 ml DCM. The combined organic phases were dried over Na₂SO₄, filtered with suction on a funnel with a fritted disk; the filtrate was washed with a total amount of 50 ml DCM. The solvent was carefully removed under reduced pressure and 300 ml hexane was added. The volume was reduced to 100 ml by evaporation, whereupon the formation of a precipitate was observed. The suspension was stirred over night at 4°C and filtered with suction on a funnel with a fritted disk. The beige filter cake was washed with a total amount of 50 ml hexane and dried at 50°C *in vacuo* for 3 h to yield 29.00 g (94.4% of theory) of the title compound as a salmon-pink solid (m.p.=
109-111°C).
¹H-NMR data (CDCl₃, 400 MHz): δ 7.37 (dd, 1H), 7.13 (dd, 1H), 6.93 (dd, 1H), 6.79 (br s, 1H), 3.05 (s, 3H).
MS m/e (%): 222 ([M-H]⁺, 100).
EA for C₇H₇ClFNO₂ calc.: C 37.59, H 3.15, N 6.26 O 14.31. Found: C 37.45, H 3.14, N 6.36 O 14.35.

### Example 1.4: Synthesis of N-(4-Chloro-3-fluoro-phenyl)-benzenesulfonamide

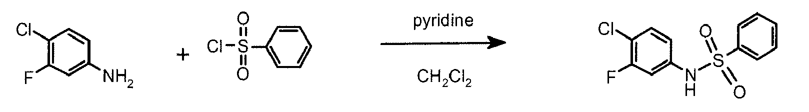

To a stirred solution of 20 g (137.4 mmol) 4-Chloro-3-fluoro aniline in 120 ml DCM was added 22.1 ml (274.8 mmol, 2 eq.) pyridine in one portion. The resulting solution was cooled to 0-3°C in an ice bath, then a solution of 19.6 ml (151.2 mmol, 1.1 eq.) benzosulfochloride in 80 ml DCM was added dropwise within 1 h, whereas the temperature was kept between 0 and 3°C. A color change from brownish over yellow and orange towards red was observed.

After 1.5 h 120 ml water was added to the reaction mixture, the organic phase was washed with 120 ml water, and the aq. phase was washed twice with total 100 ml DCM. The combined organic phases were dried over Na₂SO₄, filtered with suction on a funnel with a fritted disk and washed with total 100 ml DCM. The solvent was carefully removed under reduced pressure, then 200 ml hexane was added. The total volume was reduced to 100 ml, whereupon the formation of a precipitate was observed. The suspension was stirred over night at 4°C, the beige precipitate was filtered off with suction on a funnel with a fritted disk, washed with a total amount of 50 ml hexane and dried at 50°C *in vacuo* for 3 h to yield 39.27 g (98.3% of theory) of the title compound as a beige solid (m.p = 114.9-116.1°C).
¹H-NMR data (CDCl₃, 400 MHz): δ 7.82 (m, 2H), 7.60 (m, 1H), 7.48 (m, 2H), 7.24 (dd, 1H), 7.01 (dd, 2H), 6.78 (ddd, 1H).
MS m/e (%): 284 ([M-H]⁺, 100).
EA for C₁₂H₉ClFNO₂S calc.: C 50.44, H 3.17, N 4.90 O 11.20. Found: C 50.46, H 3.21, N 4.92 O 11.28.

### Example 1.5: Synthesis of (3,4-Difluoro-phenyl)-carbamic acid methyl ester

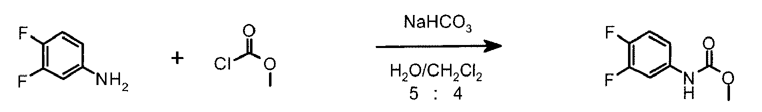

To a stirred light brown solution of 10.0 g (77.5 mmol) 3,4-Difluoro aniline in 80 ml DCM was added rapidly a solution of 6.84 g (81 mmol, 1.05 eq.) NaHCO₃ in 100 ml ofwater. To the resulting biphasic mixture was added under vigorous stirring 8.9 ml (116 mmol, 1.5 eq.) methyl chloroformate over a period of 30 min. The light brown reaction mixture was stirred for 4h at RT. The organic phase was separated and washed twice with a total amount of 50 ml water, and the combined aqueous phases were washed twice with a total amount of 100 ml DCM. The combined organic phases were dried over 2 g Na₂SO₄ and filtered with suction on a funnel with a fritted disk. The cake was washed in total with 50 ml DCM. The DCM was partially removed under reduced pressure to about 1/3 of its original volume and then 120 ml heptane was added dropwise under stirring within 30 min, whereby the product precipitated. From this suspension, DCM was completely removed under reduced pressure. Finally, the suspension was stirred in an ice bath for 1h, the crystals were filtered off with suction on a funnel with a fritted disk and washed with three 10 ml portions, in total with 30 ml ice cold heptane. The wet light brown crystals were dried at 50°C *in vacuo* to yield 13.54 g (93.4% of theory) of the title compound as light brown crystals (m.p. = 114.8-115.4°C).
¹H-NMR data (CDCl₃, 300 MHz): δ 7.43 (dd, 1H), 7.07 (dd, 1H,), 6.95 (m, 1H), 6.60 (br s, 1H), 3.78 (s, 3H).
MS m/e (%): 188 ([M+H]⁺, 100).

### Example 1.6: Synthesis of (3-Fluoro-4-methoxyphenyl)-carbamic acid methyl ester

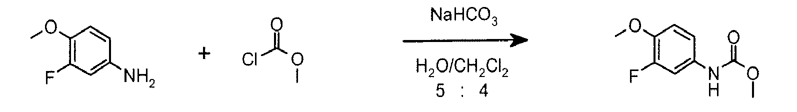

To a stirred light brown solution of 10.0 g (49.6 mmol) 3-Fluoro-4-methoxy aniline in 56 ml DCM was added rapidly a solution of 4.38 g (52 mmol, 1.05 eq.) NaHCO₃ in 70 ml of water. To the resulting biphasic mixture was added under vigorous stirring 5.72 ml (74.4 mmol, 1.5 eq.) methyl chloroformate over a period of 30 min. The light brown reaction mixture was stirred for 1.5h at RT.

The organic phase was separated and washed twice with a total amount of 50 ml water, and the combined aqueous phases were washed twice with a total amount of 100 ml DCM. The combined organic phases were dried over 2 g Na₂SO₄ and filtered with suction on a funnel with a fritted disk. The cake was washed in total with 50 ml DCM. The DCM was partially removed under reduced pressure to about 1/3 of its original volume and then 70 ml heptane was added dropwise under stirring within 30 min, whereby the product precipitated. From this suspension, DCM was completely removed under reduced pressure. Finally, the suspension was stirred in an ice bath for 1h, the crystals were filtered off with suction on a funnel with a fritted disk and washed with three 10 ml portions, in total with 30 ml ice cold heptane. The wet off-white crystals were dried at 50°C *in vacuo* to yield 9.65 g (97.7% of theory) of the title compound as off-white crystals (m.p. = 96.1-96.5°C)
¹H-NMR data (CDCl₃, 300 MHz): δ 7.29 (m, 1H), 6.98 (m, 1H,), 6.89 (dd, 1H), 6.44 (br s, 1H), 3.86 (s, 3H), 3.77 (s, 3H).
MS m/e (%): 200 ([M+H]⁺, 100).

### Example 1.7: Synthesis of (4-Bromo-3-fluoro-phenyl)-carbamic acid methyl ester

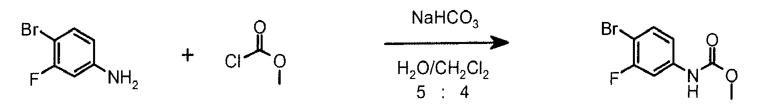

To a stirred beige solution of 10.0 g (52.6 mmol) 4-Bromo-3-fluoro aniline in 80 ml DCM was added rapidly a solution of 4.64 g (55.3 mmol, 1.05 eq.) NaHCO₃ in 100 ml of water. To the resulting biphasic mixture was added under vigorous stirring 6.07 ml (78.9 mmol, 1.5 eq.) methyl chloroformate over a period of 30 min. The beige reaction mixture was stirred for 4.5h at RT. The organic phase was separated and washed twice with a total amount of 50 ml water, and the combined aqueous phases were washed twice with a total amount of 100 ml DCM. The combined organic phases were dried over 2 g Na₂SO₄ and filtered with suction on a funnel with a fritted disk. The cake was washed in total with 50 ml DCM. The DCM was partially removed under reduced pressure to about 1/3 of its original volume and then 70 ml heptane was added dropwise under stirring within 30 min, whereby the product precipitated. From this suspension, DCM was completely removed under reduced pressure. Finally, the suspension was stirred in an ice bath for 1h, the crystals were filtered off with suction on a funnel with a fritted disk and washed with three 10 ml portions, in total with 30 ml ice cold heptane. The wet off-white crystals were dried at 50°C *in vacuo* to yield the product 12.30 g (94.3% of theory) of the title compound as off-white crystals (m.p. = 124.7-126.4°C)
¹H-NMR data (CDCl₃, 300 MHz): δ 7.44 (m, 2H), 6.94 (m, 1H,), 6.62 (br s, 1H), 3.79 (s, 3H). MS m/e (%): 250 ([M+H]⁺, 100), 248 (M⁺, 65).

### Example 1.8: Synthesis of (3-fluoro-4-methyl-phenyl)-carbamic acid methyl ester

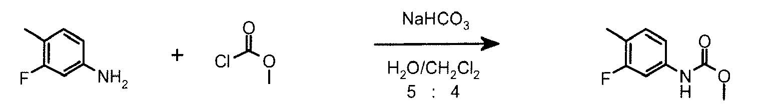

To a stirred light brown solution of 10.0 g (80 mmol) 3-Fluoro-4-methyl aniline in 80 ml DCM was added rapidly a solution of 7.05 g (84 mmol, 1.05 eq.) NaHCO₃ in 100 ml of water. To the resulting biphasic mixture was added under vigorous stirring 9.22 ml (120 mmol, 1.5 eq.) methyl chloroformate over a period of 30 min. The light brown reaction mixture was stirred for 4.5h at RT.

The organic phase was separated and washed twice with a total amount of 50 ml water, and the combined aqueous phases were washed twice with a total amount of 100 ml DCM. The combined organic phases were dried over 5 g Na₂SO₄ and filtered with suction on a funnel with a fritted disk. The cake was washed in total with 50 ml DCM. The DCM was partially removed under reduced pressure to about 1/3 of its original volume and then 70 ml heptane was added dropwise under stirring within 30 min, whereby the product precipitated. From this suspension, DCM was completely removed under reduced pressure. Finally, the suspension was stirred in an ice bath for 1h, the crystals were filtered off with suction on a funnel with a fritted disk and washed with three 10 ml portions, in total with 30 ml ice cold heptane. The wet off-white crystals were dried at 50°C *in vacuo* to yield the product 14.20 g (92.0% of theory) of the title compound as off-white crystals (m.p. = 84.8-85.1°C)
¹H-NMR data (CDCl₃, 300 MHz): δ 7.23 (m, 1H), 7.08 (dd, 1H), 6.91 (dd, 1H), 6.57 (br s, 1H), 3.77 (s, 3H), 2.21 (s, 3H).
MS m/e (%): 184 ([M+H]⁺, 100).

### Example 2.1: Synthesis of (4-Chloro-5-fluoro-2-iodo-phenyl)-carbamic acid methyl ester: I₂/NaIO₄

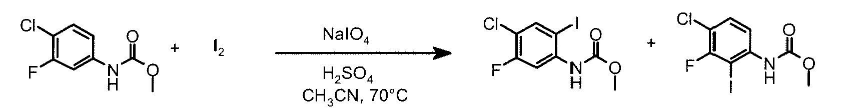

To a stirred solution of 50.0 g (243 mmol) (4-Chloro-3-fluoro-phenyl)-carbamic acid methyl ester in 300 ml MeCN were added under argon 31.2 g (122 mmol, 0.5 eq.) iodine and 15.8 g (73 mmol, 0.3 eq.) sodium periodate. The resulting the dark red suspension was heated to 70°C in an oil bath. As soon as the temperature was reached a solution of 7.4 ml (134 mmol, 0.55 eq.) sulfuric acid in 12.5 ml MeCN was added dropwise during 15 min (sulfuric acid was added dropwise to MeCN). The resulting dark red reaction mixture (suspension) was stirred at 70°C for 4 h until full conversion was achieved.

The reaction mixture was cooled to ambient temperature and quenched by dropwise addition of 500 ml Na₂SO₃ solution (25%) within 20 min, whereupon the product quantitatively precipitated and the dark red color of the reaction mixture changed to pale yellow. The suspension was stirred for 30 min in an ice bath, and the precipitate was filtered off with suction on a funnel with a fritted disk, washed several times with 100 ml water and dried at 50°C *in vacuo* to give 95 g of the crude product as white crystals with a light yellow coloration.

The crude product was suspended in 150 ml Na₂SO₃ solution (10%) and stirred vigorously for 30 min. The precipitate was filtered off with suction on a funnel with a fritted disk, and washed with about 200 ml of water. The crystals were dried at 50°C *in vacuo* to yield 75.8 g (90.6% of theory) of the title compound (96:4 mixture with its structure isomer) as white crystals (m.p.= 83-85°C). ¹H-NMR data (CDCl₃, 400 MHz): δ 8.05 (d, 1H), 7.76 (d, 1H), 6.98 (br s, 1H), 3.82 (s, 3H).
MS m/e (%): 329 (M⁺, 49), 202.0 ([M-I]⁺, 100).
EA for C₈H₆ClFINO₂ calced.: C 29.16, H 1.84, N 4.25, O 9.71. Found: C 29.10, H 1.72, N 4.46, O 9.96.

In a similar manner the reactions in Table 2.1 were performed.

**Table 2.1:**

| Solvent | T [°C] | t [h] | Conversion*^{c)}* [%] | Selectivity **2-I/6-I***^{c)}* | Isol. Yield [%] |
|---|---|---|---|---|---|
| MeCN*^{a)}* | 80 | 2 | >99 | 95.5:4.5 | 93 |
| MeCN*^{a)}* | 70 | 3.75 | >99 | 95:5 | 91 |
| MeCN *^{a)}* | 60 | 21 | >99 | 95:5 | 91 |
| AcOEt *^{a)}* | 80 | 4 | >99 | 96:4 | 92 |
| AcOH *^{b)}* | 65 | 1 | >99 | 96:4 | 82 |
| MeOH *^{b)}* | 75 | 23 | >99 | 91:9 | 84 |

| | | | | | |
|---|---|---|---|---|---|
| *^{a)}* Conditions: I₂ (0.5 eq.), NaIO₄ (0.3 eq.), H₂SO₄ (0.55 eq.). *^{b)}* conditions: I₂ (0.8 eq.), NaIO₄ (0.4 eq.), H₂SO₄ (0.55 eq.). *^{c)}* Determined by HPLC-analysis. | | | | | |

### Example 2.2: Synthesis of (4-Chloro-5-fluoro-2-iodo-phenyl)-carbamic acid methyl ester: I₂/H₅IO₆

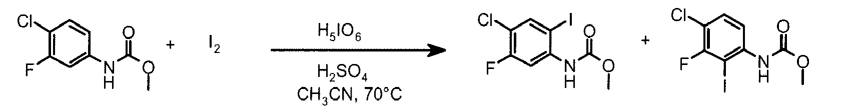

To a stirred solution of 5.0 g (24.32 mmol) (4-Chloro-3-fluoro-phenyl)-carbamic acid methyl ester in 300 ml MeCN were added under argon 246 µl (2.43 mmol, 0.1 eq.) sulfuric acid, 1.6 g (7.30 mmol, 0.3 eq.) periodic acid and 6.2 g (24.32 mmol, I eq.) iodine and the resulting dark red suspension was heated to 70°C in an oil bath. After 6.5 h 246 µl (2,43 mmol, 0.1 eq.) sulfuric acid were added and the reaction mixture was stirred overnight (20 h in total) at 50°C to achieve full conversion.

The reaction mixture was cooled to ambient temperature and was poured on a solution of 5.5 g (43.64 mmol, 1.79 eq.) Na₂SO₃ solution in 130 ml water whereupon the product quantitatively precipitated and the dark red colour of the reaction mixture changed to a pale yellow. The suspension was stirred for 30 min in an ice bath, and the precipitate was filtered off with suction on a funnel with a fritted disk, washed several times with 20 ml water and dried at 50°C *in vacuo* to give 7.75 g (92.8% of theory) of the title compound (96:4 mixture with its structure isomer) as white crystals.

### Example 2.3: Synthesis of (4-Chloro-5-fluoro-2-iodo-phenyl)-carbamic acid methyl ester: NIS

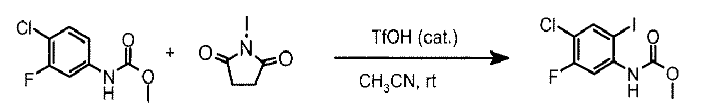

To a stirred solution of 13.0 g (63.85 mmol) (4-Chloro-3-fluoro-phenyl)-carbamic acid methyl ester in 120 ml MeCN were added under nitrogen 15.8 g (70.24 mmol, 1.1 eq.) NIS and 0.56 ml (6.39 mmol, 0.1 eq.) TfOH and the resulting solution was stirred overnight at ambient temperature. The reaction mixture was poured on 120 ml NaHCO₃ solution (sat.) and was extracted with a total amount of 450 ml AcOEt. The combined organic phases were extracted with 200 ml Na₂SO₃ solution (1N) whereas the water phase was washed with 100 ml AcOEt. The combined organic phases were dried over magnesium sulfate and filtered with suction on a funnel with a fritted disk and dried *in high vacuo* to give 21.00 g (95% of theory) of the title compound as a brown powder.

### Example 2.4: Synthesis of (4-Chloro-5-fluoro-2-iodo-phenyl)-carbamic acid methyl ester: DIH

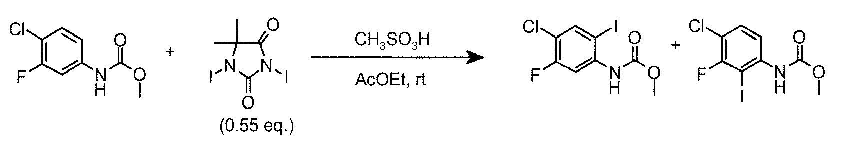

To a stirred solution of 15.0 g (72.94 mmol) (4-Chloro-3-fluoro-phenyl)-carbamic acid methyl ester in 75 ml AcOEt were added successively 9.6 ml (146 mmol, 2 eq.) methanesulfonic acid and 15.6 g (40.12 mmol, 0.55 eq.) DIH. A slight temperature increase from 25°C to 28-30°C was observed; the mixture was stirred for 1.5 h at ambient temperature.

The reaction was quenched with a solution of 28.3 g (161 mmol, 2.2 eq.) ascorbic acid in 50 ml water, stirred for 1 h and diluted with 50 ml water and 75 ml AcOEt. The aqueous phase was separated and washed with 75 ml AcOEt, the organic phase was washed with 50 ml water, the combined organic phases were dried over Na₂SO₄, filtered with suction on a funnel with a fritted disk and evaporated to dryness to yield 27.05 g of the crude product red solid.

The crude product was dissolved in 60 ml hot MeOH (refluxing) and 20 ml water were added dropwise under stirring to the hot solution whereupon the product precipitated. The suspension was stirred for 1 h in an ice bath, the white precipitate was filtered off with suction on a funnel with a fritted disk, washed thoroughly with a total amount of 30 ml water and dried at 50°C *in vacuo* (20 mbar) to yield 23.84 g (95.5% of theory) of the title compound (97:3 mixture with its structure isomer) as white crystals.

In a similar manner the reactions in Table 2.2 were performed.

**Table 2.2:**

| R | Scale (g) | Solvent | Acid (eq.) | DIH (eq.) | Selectivity (%)*^{a}* | Yield (%) | MS m/e (%): |
|---|---|---|---|---|---|---|---|
| CO₂Me | 4 | AcOH | CH₃SO₃H (2) | 0.5 | 97:3 | 94 | |
| CO₂Me | 15 | AcOEt | TfOH (2) | 0.5 | 95:5 | 92 | |
| CO₂Et | 5 | AcOEt | TfDH (2) | 0.5 | 97:3 | 89 | 343 (M⁺, 91) |
| CO₂*i*-Pr | 5 | AcOEt | TfOH (2) | 0.5 | 98:2 | 89.5 | 357 (M⁺, 80) |
| CO₂(-)-Menthyl | 1 | AcOEt | TIDH (2) | 0.5 | 95.6:4.4 | 83 | 453 (M⁺, 39) |

| | | | | | | | |
|---|---|---|---|---|---|---|---|
| Typical Reaction time 1-2 h. *^{a}* Determined by HPLC. | | | | | | | |

### Example 2.5: Synthesis of (4-Chloro-3-fluoro-2-iodo-phenyl)-carbamic acid methyl ester

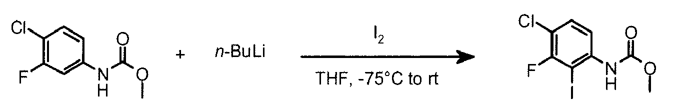

To a stirred solution of 10.0 g (49.12 mmol) (4-Chloro-3-fluoro-phenyl)-carbamic acid methyl ester in 130 ml THF was added under argon at -75°C over 30 min 76.8 ml (123 mmol, 2.5 eq.) *n-*BuLi. The resulting solution was stirred for 1.5 h whereupon its color turned from red over colorless to green and finally yellow. To this yellow solution a red suspension of 37.8 g (147 mmol, 3 eq.) iodine in 160 ml THF was added dropwise, the black reaction mixture was allowed to warm to 0-5°C and stirred for 2.5 h at this temperature. Finally, the ice bath was removed and the reaction mixture was stirred over night at ambient temperature.

To the black suspension was added with stirring a solution of 22.0 g (174 mmol, 3.55 eq.) Na₂SO₃ in 100 ml of water. A biphasic yellow mixture was obtained. The water phase was separated and washed with 100 ml TBME whereas the organic phase was washed with 100 ml sat. sodium chloride solution. The combined organic phases were dried over Na₂SO₄, filtered over a glass funnel with a fritted disk. The filter cake was washed with a total amount of 50 ml TBME. Evaporation of the combined filtrates gave 11.9 g of the crude product.

To a solution of the crude product in 200 ml MeOH at reflux was added dropwise 60 ml water, whereupon the product precipitated. The heating bath was removed; the mixture was stirred at ambient temperature for 1 h and in an ice bath for 1 h. The beige precipitate was filtered on a glass funnel with a fritted disk, washed with water and dried at 50°C *in vacuo* until weight constancy to yield 14.5 g (88.0% of theory) of the title compound as off-white crystals (m.p.= 137-139°C).
¹H-NMR data (CDCl₃, 400 MHz): δ 7.88 (d, 1H,), 7.76 (dd, 1H), 7.04 (br s, 1H), 3.82 (s, 3 H). MS m/e (%): 329 (M⁺, 66), 202.0 ([M-I]⁺, 100).

### Example 2.6: Synthesis of (5-Chloro-4-fluoro-2-iodo-phenyl)-carbamic acid methyl ester

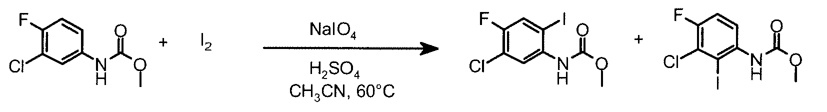

To a stirred solution of 20.0 g (243 mmol) (3-Chloro-4-fluoro-phenyl)-carbamic acid methyl ester in 120 ml MeCN were added under argon 12.5 g (48.63 mmol, 0.5 eq.) iodine and 6.3 g (29.18 mmol, 0.3 eq.) sodium periodate. The resulting the dark red suspension was heated to 60°C in an oil bath. Then a solution of 3.0 ml (53.4 mmol, 0.55 eq.) sulfuric acid in 5.0 ml MeCN was added dropwise during 15 min. The resulting dark red reaction mixture (suspension) was stirred at 60°C for 25 h followed by the addition of 2.5 g (9.73 mmol, 0.1 eq.) iodine, 2.0 g (9.73 mmol, 0.1 eq.) sodium periodate and 537 µl (9.73 mmol, 0.1 eq.) sulfuric acid. The reaction mixture was stirred for an additional 28 h at 60°C (53 h in total) and then cooled to ambient temperature and quenched by dropwise addition of 100 ml Na₂SO₃_solution (25%) within 20 min, whereupon the product precipitated and the dark red color of the reaction mixture changed to pale yellow. The suspension was stirred for 30 min, and the precipitate was filtered off with suction on a funnel with a fritted disk, washed several times with 20 ml water and dried at 50°C *in vacuo* until weight constancy to give 34 g of the crude product as white crystals with a light yellow coloration.

The crude product was suspended in 100 ml Na₂SO₃ solution (10%) and stirred vigorously for 30 min. The precipitate was filtered off with suction on a funnel with a fritted disk, and washed with about 200 ml of water. The crystals were dried under at 50°C *in vacuo* until weight constancy to yield 24.2 g (65.7% of theory) of the title compound (87:13 mixture with its structure isomer) as white crystals (m.p.= 99-101.5°C).
¹H-NMR data (CDCl₃, 400 MHz): δ 8.16 (d, 1H), 7.54 (d, 1H), 6.82 (br s, 1H), 3.81 (s, 3H). MS m/e (%): 329 (M⁺, 50), 202 ([M-I]⁺, 100).

### Example 2.7: Synthesis of N-(4-Chloro-5-fluoro-2-iodo-phenyl)-methanesulfonamide

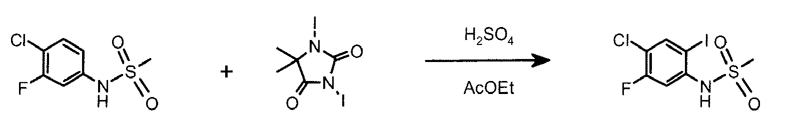

To a solution of 5 g (22.36 mmol) N-(4-Chloro-3-fluoro-phenyl)-methanesulfonamide in 100 ml AcOEt was added under stirring 2.4 ml (44.72 mmol, 2 eq.) sulfuric acid. A small temperature increase to 28°C was observed. To this solution, 4.3 g (17.5 mmol, 0.5 eq.) DIH was added in one portion whereupon the mixture turned to a yellow suspension. The mixture was stirred for 1 h and was then heated for 2.5 h at 40°C and for 1 h at 50°C.

After cooling to ambient temperature, the red mixture was quenched with 5.6 g (44.72 mmol, 2 eq.) Na₂SO₃ in 30 ml water and stirred for 30 min. The organic phase was washed with 30 ml water, the aqueous phase was extracted with 50 ml AcOEt. The combined organic phases were dried over Na₂SO₄, filtered and the solvent was carefully removed under reduced pressure, to yield the 10.05 g of crude product as a beige solid.

The crude product was dissolved under stirring in 25 ml MeOH at reflux and to the hot mixture 15 ml water was added dropwise whereupon precipitation started. The mixture was cooled to ambient temperature and then stirred for 3 h at 4°C. The precipitate was filtered off with suction on a funnel with a fritted disk, washed with a total amount of 25 ml ice-cold mixture of water/MeOH (2:1) and dried at 50°C *in vacuo* to weight constancy to give 6.28 g (79.6% of theory) of the title compound (99:1 mixture with its structure isomer) as white crystals (m.p.= 122.6-124.5°C).
¹H-NMR data (CDCl₃, 400 MHz): δ 7.84 (d, 1H), 7.53 (d, 1H), 6.64 (br s, 1H), 3.04 (s, 3H). MS m/e (%): 348 ([M-H]⁺, 100).

### Example 2.8: Synthesis of N-(4-Chloro-5-fluoro-2-iodo-phenyl)-benzenesulfonamide

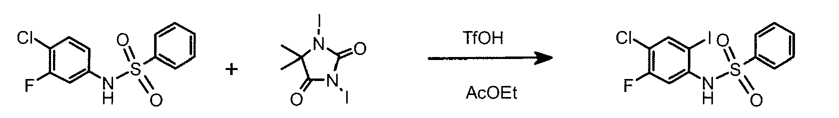

To a stirred solution of 10 g (35.0 mmol) N-(4-Chloro-3-fluoro-phenyl)-benzenesulfonamide in 200 ml AcOEt was added dropwise 6.2 ml (70.0 mmol, 2 eq.) TfOH. A small temperature increase to 28°C was observed. To this solution, 6.8 g (17.5 mmol, 0.5 eq.) DIH was added in one portion whereupon the mixture turned orange.

The mixture was stirred for 4 h at ambient temperature and then was quenched with a solution of 15.5 g (87.5 mmol, 2.5 eq.) ascorbic acid in 125 ml water. The resulting reaction mixture was stirred for 30 min at ambient temperature, then the phases were separated, the aq. phase was extracted with 125 ml AcOEt, whereas the organic phase was washed with 125 ml water. The combined organic phases were dried over Na₂SO₄, filtered with suction on a funnel with a fritted disk and evaporated to dryness to yield 16.75 g of the crude product of the title compound as a light brown solid.

To a stirred solution of the crude product in 50 ml MeOH was added at reflux 30 ml water dropwise whereupon precipitation started. The oil bath was removed, the mixture was allowed to cool to ambient temperature and was then stirred overnight at 4°C in the fridge. The precipitate was filtered off with suction on a funnel with a fritted disk, washed with a total amount of 20 ml ice-cold mixture of water/MeOH (2:1) and dried at 50°C *in vacuo* for 4 h to weight constancy to give 12.89 g (87.1% of theory) of the title compound (98:2 mixture with its structure isomer) as beige crystals (m.p.= 122.5-124.5°C).
¹H-NMR data (CDCl₃, 400 MHz): δ 7.78 (m, H), 7.67 (d, 1H), 6.62-7.46 (m, 4H), 6.80 (br s, 1H). MS m/e (%): 410 ([M-H]⁺, 100).

### Example 2.9: Synthesis of (4,5-difluoro-2-iodo-phenyl)-carbamic acid methyl ester

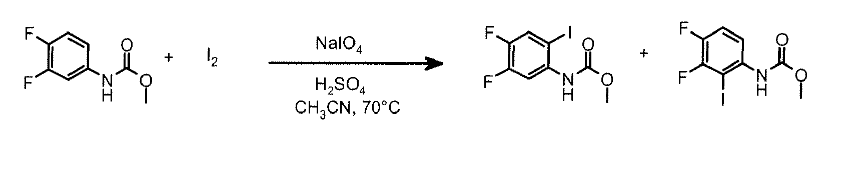

To a stirred solution of 10.0 g (53.5 mmol) (3,4-difluoro-phenyl)-carbamic acid methyl ester in 60 ml MeCN were added under argon 5.44 g (27.8 mmol, 0.5 eq.) iodine and 3.43 g (16 mmol, 0.3 eq.) sodium periodate. The resulting dark red suspension was heated to 70°C in an oil bath.

As soon as the temperature was reached a solution of 1.57 ml (29 mmol, 0.55 eq.) sulfuric acid in 5 ml MeCN was added dropwise during 15 min. The resulting dark red reaction mixture (suspension) was stirred at 70°C for 1 h and 40 min before 1.35 g (64 mmol, 0.12 eq.) iodine, 0.68 g (3 mmol, 0.06 eq.) sodium periodate and 0.314 ml (59 mmol, 0.11 eq.) sulfuric acid was added. After another 30 min full conversion was achieved.

The reaction mixture was cooled to ambient temperature and quenched by dropwise addition of 200 ml Na₂SO₃ solution (25%) within 20 min, whereupon the product quantitatively precipitated and the dark red color of the reaction mixture changed to pale yellow. The suspension was stirred for 30 min in an ice bath, and the precipitate was filtered off with suction on a funnel with a fritted disk, washed several times with 50 ml water and dried at 50°C *in vacuo* until weight constancy to give 18.54 g of the crude product as beige crystals.

The crude product was suspended in 100 ml ofwater and stirred vigorously for 1h. The precipitate was filtered off with suction on a funnel with a fritted disk, and washed with about 200 ml of water. The crystals were dried at 50°C *in vacuo* until weight constancy to yield 15.47 g (90% of theory) of the title compound (97:3 mixture with its structure isomer) as beige crystals (m.p.= 57.2-58.8°C)
¹H-NMR data (CDCl₃, 300 MHz): δ 8.05 (dd, 1H), 7.55 (dd, 1H), 6.90 (br s, 1H), 3.81 (s, 3H). MS m/e (%): 312 ([M-H]⁺, 100).

### Example 2.10: Synthesis of (5-Fluoro-2-iodo-4-methoxy-phenyl)-carbamic acid methyl ester

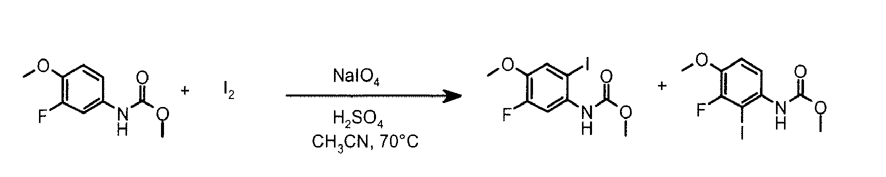

To a stirred solution of 9.4 g (47 mmol) (3-Fluoro-4-methoxy-phenyl)-carbamic acid methyl ester in 56 ml MeCN were added under argon 5.99 g (23.6 mmol, 0.5 eq.) iodine and 3.03 g (14 mmol, 0.3 eq.) sodium periodate. The resulting dark red suspension was heated to 70°C in an oil bath.

As soon as the temperature was reached a solution of 1.39 ml (26 mmol, 0.55 eq.) sulfuric acid in 2.4 ml MeCN was added dropwise during 15 min. The resulting dark red reaction mixture (suspension) was stirred at 70°C until full conversion was achieved (3h).

The reaction mixture was cooled to ambient temperature and quenched by dropwise addition of 200 ml Na₂SO₃ solution (25%) within 20 min, whereupon the product precipitated and the dark red color of the reaction mixture changed to pale yellow. The suspension was stirred for 30 min in an ice bath, and the precipitate was filtered off with suction on a funnel with a fritted disk, washed several times with 50 ml water and dried at 50°C *in vacuo* to give the crude product as beige crystals.

The crude product was suspended in 100 ml of water and stirred vigorously for 1h. The precipitate was filtered off with suction on a funnel with a fritted disk, and washed with about 200 ml of water. The crystals were dried at 50°C *in vacuo* to yield 10.7 g (70% of theory) of the title compound (99.7:0.3 mixture with its structure isomer) as beige crystals (m.p.= 124.5-124.9°C)
¹H-NMR data (CDCl₃, 300 MHz): δ 7.85 (d, 1H), 7.29 (d, 1H), 6.75 (br s, 1H), 3.85 (s, 3H), 3.80 (s, 3H).
MS m/e (%): 326 ([M+H]⁺, 59), 199 ([M-I]⁺, 100).

### Example 2.11: Synthesis of (4-Bromo-5-fluoro-2-iodo-phenyl)-carbamic acid methyl ester

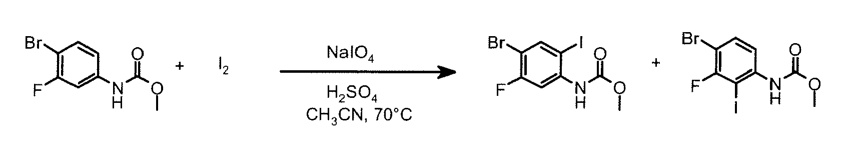

To a stirred solution of 10 g (40 mmol) (4-Bromo-3-fluoro-phenyl)-carbamic acid methyl ester in 60 ml MeCN were added under argon 5.12 g (20.2 mmol, 0.5 eq.) iodine and 2.59 g (12.1 mmol, 0.3 eq.) sodium periodate. The resulting dark red suspension was heated to 70°C in an oil bath. As soon as the temperature was reached a solution of 1.2 ml (22 mmol, 0.55 eq.) sulfuric acid in 2.5 ml MeCN was added dropwise during 15 min. The resulting dark red reaction mixture (suspension) was stirred at 70°C until full conversion was achieved (4h).

The reaction mixture was cooled to ambient temperature and quenched by dropwise addition of 100 ml Na₂SO₃ solution (25%) within 20 min, whereupon the product precipitated and the dark red color of the reaction mixture changed to pale yellow. The suspension was stirred for 30 min in an ice bath, and the precipitate was filtered off with suction on a funnel with a fritted disk, washed several times with 50 ml water and dried at 50°C *in vacuo* to give 13.5 g (86% of theory) of the title compound (96:4 mixture with its structure isomer) as white crystals (m.p.= 103.8-104.1°C)
¹H-NMR data (CDCl₃, 300 MHz): δ 8.04 (d, 1H), 7.90 (d, 1H), 7.02 (br s, 1H), 3.82 (s, 3H). MS m/e (%): 372 ([M-2H]⁺, 100).

### Example 2.12: Synthesis of (5-Fluoro-2-iodo-4-methyl-phenyl)-carbamic acid methyl ester

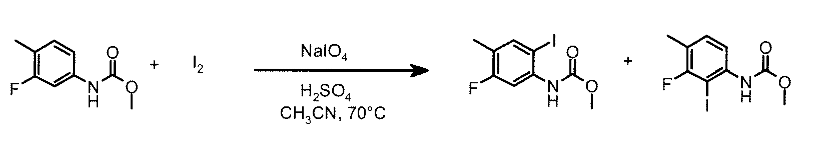

To a stirred solution of 11 g (60 mmol) (3-Fluoro-4-methyl-phenyl)-carbamic acid methyl ester in 66 ml MeCN were added under argon 7.62 g (30.3 mmol, 0.5 eq.) iodine and 3.85 g (12.1 mmol, 0.3 eq.) sodium periodate. The resulting dark red suspension was heated to 70°C in an oil bath. As soon as the temperature was reached a solution of 1.77 ml (22 mmol, 0.55 eq.) sulfuric acid in 2.75 ml MeCN was added dropwise during 15 min. The resulting dark red reaction mixture (suspension) was stirred at 70°C until full conversion was achieved (3h).

The reaction mixture was cooled to ambient temperature and quenched by dropwise addition of 110 ml Na₂SO₃ solution (25%) within 20 min and the dark red color of the reaction mixture changed to pale yellow. The suspension was stirred for 30 min in an ice bath whereupon the product precipitated. The precipitate was filtered off with suction on a funnel with a fritted disk, washed several times with 50 ml water and dried at 50°C *in vacuo* to give 13.9 g (74% of theory) of the title compound (99.3:0.7 mixture with its structure isomer) as off-white crystals (m.p.= 85.9-86.3°C).
¹H-NMR data (CDCl₃, 300 MHz): δ 7.83 (d, 1H), 7.55 (dd, 1H), 6.96 (br s, 1H), 3.80 (s, 3H). MS m/e (%): 310 ([M+H]⁺, 74), 183 ([M-I]⁺, 100).

### Example 2.13: Synthesis of (4-Bromo-2-iodo-5-methyl-phenyl)-carbamic acid methyl ester

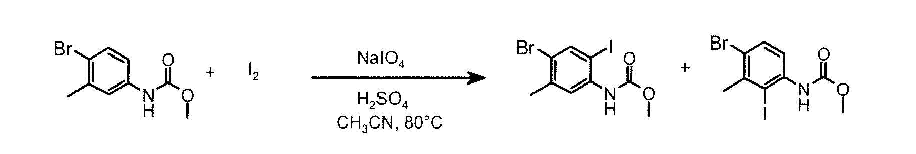

To a stirred solution of 2.69 g (11 mmol) (4-Bromo-3-methyl-phenyl)-carbamic acid methyl ester in 40 ml MeCN were added under argon 1.40 g (5.5 mmol, 0.5 eq.) iodine, 0.708 g (3.3 mmol, 0.3 eq.) sodium periodate and 0.31 ml (5.5 mmol, 0.5 eq.) sulfuric acid. The resulting dark red suspension was heated to 80°C in an oil bath and stirred at this temperature until full conversion was achieved (3.5h).

The reaction mixture was cooled to ambient temperature and quenched by dropwise addition of 55 ml Na₂SO₃ solution (5%) within 20 min and the dark red color of the reaction mixture changed to pale yellow. The suspension was stirred for 30 min in an ice bath whereupon the product precipitated. The precipitate was filtered off with suction on a funnel with a fritted disk, washed several times with 10 ml water and dried at 45°C *in vacuo* to give 3.96 g (87% of theory) of the title compound (9:1 mixture with its structure isomer) as light brown crystals (m.p.= 133.0-135.8°C)
¹H-NMR data (CDCl₃, 300 MHz): δ 7.97 (s, 1H), 7.87 (s, 1H), 6.90 (br s, 1H), 3.80 (s, 3H), 2.37 (s, 3H).
MS m/e (%): 370 ([M]⁺, 73), 243 ([M-H-I]⁺, 100).

### Example 3.1: Synthesis of (4-Chloro-5-fluoro-2-trimethylsilanylethynyl-phenyl)-carbamic acid methyl ester

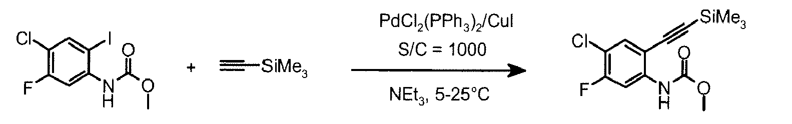

A mixture of 150.3 ml NEt₃, 42.6 mg (0.061 mmol, S/C = 1000) PdCl₂(PPh₃)₂ and 16.5 mg (0.085 mmol, 1.4 eq. vs. Pd) copper iodide were heated under argon at reflux for 30 min to form the catalyst. The resulting yellow catalyst solution was cooled to 0-5°C in an ice bath and then 20 g (60.68 mmol) (4-Chloro-5-fluoro-2-iodo-phenyl)-carbamic acid methyl ester and 8.6 ml (60.68 mmol) trimethylsilylacetylene were added to give a brownish suspension. The mixture was stirred for 10 min at 0-5°C and then the ice bath was removed, such that the reaction mixture slowly reached ambient temperature.

After full conversion was reached, the precipitate (NEt₃I) was filtered off with suction on a funnel with a fritted disk, washed with a total amount of 50 ml toluene. Evaporation of the solvent to dryness afforded 19.1 g of the crude product as light brownish solid.

The crude product was dissolved in 40 ml ethanol at reflux. After removal of the heating bath, the resulting solution was allowed to cool to ambient temperature under stirring, whereupon white crystals started to precipitate. The crystallization was completed by stirring the suspension in an ice bath (0-5 °C) for 1 h. The crystals were filtered off with suction on a funnel with a fritted disk, washed with 5 ml ice cold ethanol and dried at 50°C *in vacuo* for 3h to give 17.3 g (93.8% of theory) of the title compound as off-white crystals (m.p.= 93-94°C).
¹H-NMR data (CDCl₃, 400 MHz): δ 8.08 (d, 1H), 7.44 (br s, 1H), 7.41 (d, 1H), 3.82 (s, 3H), 0.28 (s, 9H).
MS m/e (%): 299 (M⁺, 82).

In a similar manner, the reactions in Table 3.1 were performed.

**Table 3.1:**

| CO₂R | Scale (g) | S/C | Yield [%] |
|---|---|---|---|
| Et | 10 | 1000 | 94 |
| *i*-Pr | 5 | 200 | 95.4 |

### Example 3.2: Synthesis of [4-Chloro-5-fluoro-2-(3-hydroxy-3-methyl-but-1-ynyl)-phenyl]-carbamic acid methyl ester

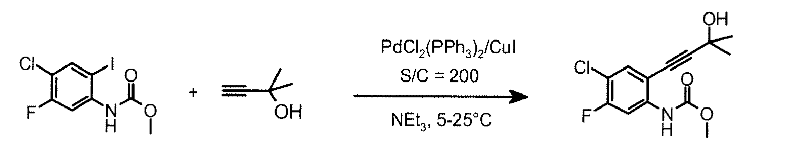

A mixture of 75.2 ml NEt₃, 106.5 mg (0.152 mmol, S/C = 200) PdCl₂(PPh₃)₂ and 35.4 mg (0.182 mmol, 1.2 eq. vs. Pd) copper iodide were heated under argon at reflux for 30 min to form the catalyst. The resulting yellow catalyst solution was cooled to 0-5°C in an ice bath, then 10 g (30.35 mmol) (4-Chloro-5-fluoro-2-iodo-phenyl)-carbamic acid methyl ester and 3.0 ml (30.35 mmol) 2-methyl-3-butyn-2-ol were added to give a brownish suspension. The mixture was stirred for 10 min at 0-5°C, then the ice bath was removed, such that the reaction mixture slowly reached ambient temperature.

After 2h full conversion was reached, the precipitate (NEt₃I) was filtered off with suction on a funnel with a fritted disk and washed with a total amount of 25 ml toluene. Evaporation of the solvent to dryness afforded 9.6 g of the crude product as light brownish solid. The crude product was filtered over silica gel with AcOEt/heptane (1:1) to yield 8.84 g of the crude product "2" as brownish oil, which solidified upon standing overnight.

The crude product "2" was digested in 100 ml heptane at reflux. After removal of the heating bath, the resulting solution was allowed to cool to ambient temperature under stirring. The crystallization was completed by stirring the suspension in an ice bath (0-5°C) for 1 h. The crystals were filtered off with suction on a funnel with a fritted disk, washed with 25 ml ice cold heptane and dried at 50°C *in vacuo* for 3h to give 7.2 g (83.0% of theory) of the title compound as off-white crystals (m.p.= 84-85°C).
¹H-NMR data (CDCl₃, 400 MHz): δ 8.06 (d, 1H), 7.38 (d, 1H), 7.31 (br s, 1H), 3.81 (s, 3H), 2.09 (s, 1H), 1.65 (s, 6H).
MS m/e (%): 285 (M⁺, 100).

### Example 4.1: Synthesis of 3-Chloro-2-fluoro-6-bis(methoxycarbonylamino)-5-trimethyl-silanylethynyl-benzoic acid methyl ester; amine: dicyclohexyl amine

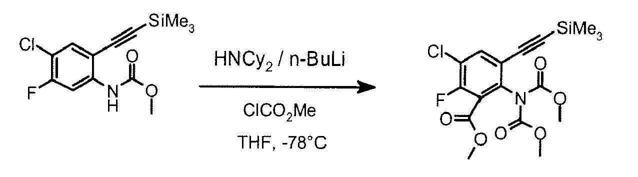

To a stirred solution of 19.42 ml (97.59 mmol, 2.25 eq.) dicyclohexylamine in 32.5 ml THF, in a vessel called "A", was added under argon dropwise at -20°C 55.54 ml (88.92 mmol, 1.6M, 2.05 eq.) *n*-BuLi solution in hexane. The resulting suspension was stirred at -20°C for 30 min.

To a solution of 13 g (43.37 mmol) (4-Chloro-5-fluoro-2-trimethylsilanyl-ethynyl-phenyl)-carbamic acid methyl ester in 195 ml THF at temperature between -70 to -74°C, under continuous stirring and argon, was slowly added the content of vessel "A" over a 30 min period via a teflon cannula. The reaction mixture was stirred for additional 1.5 h at -70°C and then a solution of 7.2 ml (91.00 mmol, 2.1 eq.) methyl chloroformate in 50.0 ml THF was added and the resulting white suspension was stirred for 1 h at the same temperature to give a yellow solution. Afterwards the solution was warmed to 0-5°C within 10 min with an ice bath and quenched with a solution of 9.4 g (49.01 mmol, 1.13 eq.) citric acid in 182 ml water. After having added 130 ml of TBME the thick suspension was stirred for 30 additional minutes. The suspension was filtered with suction on a funnel with a fritted disk, the filtrate extracted with TBME, dried over Na₂SO₄, filtered with suction on a funnel with a fritted disk. The solvent was removed under reduced pressure to give 19.2 g of the crude product as a brown oil, which solidified upon standing.

The crude product was dissolved in 50 ml methylcyclohexane at reflux under stirring and then cooled to ambient temperature such that white crystals precipitated. The suspension was stirred in an ice bath (0-5°C) for 1 h, the precipitate was filtered off with suction on a funnel with a fritted disk, dried at 50°C *in vacuo* to give 15.7 g (87.6% of theory) of the title compound as white crystals (m.p.= 99-100°C).
¹H-NMR data (CDCl₃, 400 MHz): δ 7.64 (d, 1H), 3.90 (s, 3H), 3.76 (s, 6H), 0.22 (s, 9H). MS m/e (%): 415 (M⁺, 30), 400 [M-CH₃]⁺, 100).
EA calc. for C₁₇H₁₉ClFNO₆Si: C 49.10, H 4.60, N 3.37. Found: C 48.94, H 4.80, N 3.28.

### Example 4.2: Synthesis of 3-Chloro-2-fluoro-6-bis(methoxycarbonylamino)-5-trimethyl-silanylethynyl-benzoic acid methyl ester; amine: diisopropyl amine

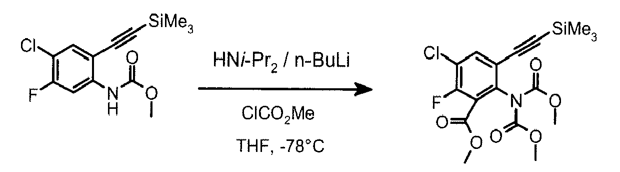

To a stirred solution of 10.64 ml (75.06 mmol, 2.25 eq.) diisopropylamine in 25 ml THF, in a vessel called "B", was added under argon dropwise at -20°C 42.72 ml (68.38 mmol, 1.6M, 2.05 eq.) n-BuLi solution in hexane. The resulting suspension was stirred at -20°C for 30 min.

To a solution of 10 g (33.36 mmol) (4-Chloro-5-fluoro-2-trimethylsilanyl-ethynyl-phenyl)-carbamic acid methyl ester in 150 ml THF at a temperature between -70 to -74°C while stirring and under argon, was slowly added the content of vessel "B" over a 30 min period via a teflon cannula. The reaction mixture was stirred for additional 1.5 h at -70°C and then a solution of 5.5 ml (70.06 mmol, 2.1 eq.) methyl chloroformate in 40.0 ml THF was added and the resulting white suspension was stirred for 1 h at the same temperature to give a yellow solution. Afterwards the solution was warmed to 0-5°C within 10 min with an ice bath and quenched with 16.7 ml (292.0 mmol, 8.75 eq.) acetic acid, and the thick suspension was stirred for 30 additional minutes in an ice bath. The suspension was filtered with suction on a funnel with a fritted disk and the filter cake was washed with TBME. The filtrate was extracted with TBME, dried over Na₂SO₄ and filtered with suction on a funnel with a fritted disk. The solvent was removed under reduced pressure to give the crude product 14.7 g of the title compound as brown oil, which solidified upon standing.

The crude product was dissolved in 35 ml methylcyclohexane at reflux under stirring and then cooled to ambient temperature such that white crystals precipitated. The suspension was stirred in an ice bath (0-5°C) for 1 h, the precipitate was filtered off with suction on a funnel with a fritted disk, washed with 7 ml methylcyclohexane and dried at 50°C *in vacuo* to give 9.45 g (68.1 % of theory) of the title compound as white crystals.

### Example 4.3: Synthesis of 3-Chloro-2-fluoro-6-bis(methoxycarbonylamino)-5-trimethyl-silanylethynyl-benzoic acid methyl ester; amine: hexamethyl disilazane

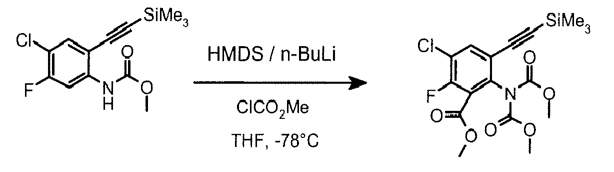

To a stirred solution of 8.14 ml (37.53 mmol, 2.25 eq.) hexamethyldisilazane in 12.5 ml THF, in a vessel called "C", was added under argon dropwise at -20°C 21.36 ml (34.19 mmol, 1.6M, 2.05 eq.) n-BuLi solution in hexane. The resulting suspension was stirred at -20°C for 30 min.

To a solution of 5 g (16.68 mmol) (4-Chloro-5-fluoro-2-trimethylsilanyl-ethynyl-phenyl)-carbamic acid methyl ester in 75 ml THF at temperature between -70 to -74°C was slowly added, under argon with constant stirring, the content of vessel "C" during 30 min via a teflon cannula. The reaction mixture was stirred for additional 1.5 h at -70°C and then a solution of 2.75 ml (35.01 mmol, 2.1 eq.) methyl chloroformate in 20 ml THF was added and the resulting white suspension was stirred for 1 h at the same temperature to give a yellow solution. Afterwards the solution was warmed to 0-5°C within 10 min with an ice bath and quenched with 8.35 ml (146.0 mmol, 8.75 eq.) acetic acid in, and the thick suspension was stirred for 30 additional minutes in an ice bath. The suspension was filtered with suction on a funnel with a fritted disk and the filter cake was washed with TBME. The filtrate was extracted with TBME, dried over Na₂SO₄ and filtered with suction on a funnel with a fritted disk. The solvent was removed under reduced pressure to give the crude product 6.9 g of the title compound as a brown oil, which solidified upon standing.

The crude product was dissolved in 15 ml methylcyclohexane at reflux under stirring and then cooled to ambient temperature such that white crystals precipitated. The suspension was stirred in an ice bath (0-5°C) for 1 h, the precipitate was filtered off with suction on a funnel with a fritted disk, washed with 3 ml methylcyclohexane and dried *in vacuo* to give 4.8 g (69.2% of theory) of the title compound as white crystals.

### Example 4.4: Synthesis of 3-Chloro-2-fluoro-6-bis(methoxycarbonylamino)-5-trimethyl-silanylethynyl-benzoic acid methyl ester; amine: tetramethyl piperidine

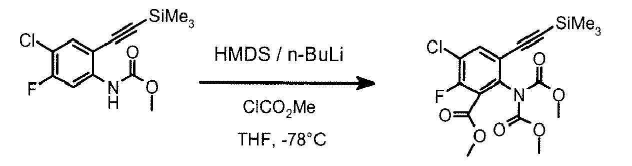

To a stirred solution of 6.34 ml (37.53 mmol, 2.25 eq.) 2,2,6,6-tetramethyl piperidine in 12.5 ml THF, in a vessel called "D", was added under argon dropwise at -20°C 21.36 ml (34.19 mmol, 1.6M, 2.05 eq.) n-BuLi solution in hexane. The resulting suspension was stirred at -20°C for 30 min.

To a solution of 5 g (16.68 mmol) (4-Chloro-5-fluoro-2-trimethylsilanyl-ethynyl-phenyl)-carbamic acid methyl ester in 75 ml THF at temperature between -70 to -74°C was slowly added, under argon and under constant stirring the content of vessel "D" during 30 min via a teflon cannula. The reaction mixture was stirred for additional 1.5 h at -70°C and then a solution of 2.75 ml (35.01 mmol, 2.1 eq.) methyl chloroformate in 20 ml THF was added and the resulting white suspension was stirred for 1 h at the same temperature to give a yellow solution.

Afterwards the solution was warmed to 0-5°C within 10 min with an ice bath and quenched with 8.35 ml (146.0 mmol, 8.75 eq.) acetic acid in, and the thick suspension was stirred for 30 additional minutes in an ice bath. The suspension was filtered with suction on a funnel with a fritted disk and the filter cake was washed with TBME. The filtrate was extracted with TBME, dried over Na₂SO₄ and filtered with suction on a funnel with a fritted disk. The solvent was removed under reduced pressure to give the crude product 7.2 g of the title compound as brown oil, which solidified upon standing.

The crude product was dissolved in 15 ml methylcyclohexane at reflux under stirring and then cooled to ambient temperature such that white crystals precipitated. The suspension was stirred in an ice bath (0-5 °C) for 1 h, the precipitate was filtered off with suction on a funnel with a fritted disk, washed with 3 ml methylcyclohexane and dried at 50°C *in vacuo* to yield 5.35 g (77.1% of theory) of the title compound as white crystals.

### Example 5.1: Synthesis of 5-Chloro-6-fluoro-1H-indole-7-carboxylic acid

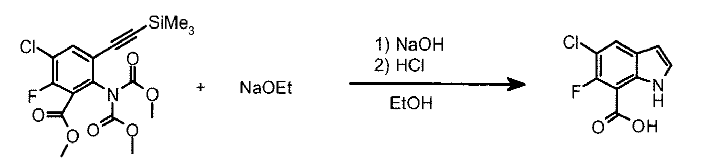

To a stirred solution of 13 g (31.26 mmol) 3-Chloro-2-fiuoro-6-bis(methoxycarbonylamino)-5-trimethyl-silanylethynyl-benzoic acid methyl ester in 160 ml ethanol was added under argon 2.7 g (37.51 mmol, 1.2 eq.) NaOEt. The resulting thin suspension was heated at 80°C for 90 min, cooled to ambient temperature and evaporated to dryness. Afterwards the brown residue was taken up in a mixture of 101.6 ml (1.1 mol, 32%, 35.1 eq.) NaOH and 94.2 ml water and the suspension was stirred at 80°C for 1 h. The precipitate was filtered with suction on a funnel with a fritted disk and washed thoroughly with water. The wet filter cake was suspended in a mixture of 60.3 ml water and 54.2 ml AcOEt. Under vigorous stirring 9.4 ml (73.15 mmol, 25%, 2.34 eq.) HCl was added, such that the pH was adjusted to 1.

The organic phase was separated and washed with water, whereas the water phase was washed with AcOEt. The combined organic phases were dried over Na₂SO₄ and filtered with suction. After addition of 135 ml toluene the AcOEt was removed under reduced pressure such that precipitation of the product started. After removal of all AcOEt, the resulting suspension was stirred at ambient temperature for 0.5 h the precipitate was filtered off with suction on a funnel with a fritted disk and washed with 10 ml toluene. The crystals were dried at 50°C *in vacuo* to yield 5.4 g (80.2% of theory) of the title compound as white crystals.
¹H-NMR data (DMSO, 400 MHz): δ 13.55 (br s, 1H), 11.25 (br s, 1H), 8.00 (d, 1H), 7.42 (dd, 1H),6.53(dd, 1H).
MS m/e (%): 212 ([M-H]⁺, 100).

EA calced. for C₉H₅ClFNO₂: C 50.61, H 2.36, N 6.56, O 14.98. Found: C 50.40, H 2.28, N 6.70, O 14.98.

In a similar manner the reactions in Table 5.1 with alternative cyclization reagents were performed.

**Table 5.1:**

| Cyclization Reagent | Solvent | Scale (g) | T[°C] | t [h] | Conversion [%]*^{a}* |
|---|---|---|---|---|---|
| NaOMe | MeOH | 0.5 | 70 | 1h | >99 |
| NaO*i*Pr | *i*-PrOH | 2.5 | 80 | 20 | >99 |
| NaO*t*Bu | *t*-BuOH | 0.5 | 80 | 6 | 60 |
| NaO*t*Bu | *i*-PrOH | 1 | 80 | 1 | >99 |
| NaO*t*Bu | THF | 0.5 | 80 | 3 | >99 |
| NaO*t*Bu | DMF | 0.5 | 100 | 2 | 90 |
| NaO*t*Bu | Toluene | 0.5 | 120 | 3 | 50 |

| | | | | | |
|---|---|---|---|---|---|
| *^{a}* Determined by HPLC. | | | | | |

### Example 5.2: Synthesis of 5-Chloro-6-fluoro-1H-indole-7-carboxylic acid

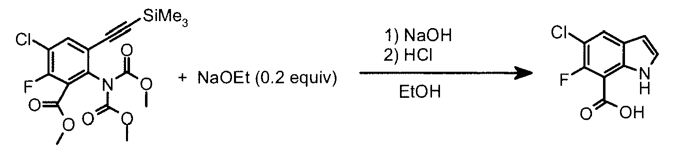

To a stirred solution of 42.4 g (102 mmol) 3-Chloro-2-fluoro-6-bis(methoxycarbonylamino)-5-trimethyl-silanylethynyl-benzoic acid methyl ester in 500 ml ethanol was added under argon 1.46 g (20.39 mmol, 0.2 eq.) NaOEt in 20 ml ethanol. The resulting brown solution was warmed to 60°C (interior temperature: 56°C), stirred at this temperature for 110 min. and quenched with 2 ml water. The reaction mixture was cooled to 45°C and the solvent was partly evaporated under reduced pressure to about 40% of the initial reaction volume, whereupon a precipitate is formed. To the resulting yellow suspension was treated with 331.2 ml (3.58 mol, 32%, 35.1 eq.) NaOH and 130.5 ml water and the suspension was heated to 80°C and stirred at this temperature for 70 min. The solvent was partly removed to eliminate all ethanol present in the reaction mixture and the suspension was cooled from 50°C to 15°C over a period of 1 h. The precipitate was filtered with suction on a funnel with a fritted disk and washed with 30 ml water. The wet filter cake was suspended in a mixture of 195.7 ml water and 260.9 ml AcOEt. Under vigorous stirring 30.7 ml (235.5 mmol, 25%, 2.31 eq.) HCl was added, such that the pH was adjusted to 1.

The organic phase was separated and the aqueous phase was washed with 65 ml water and the combined organic phases were extracted with 65 ml AcOEt. The combined organic phases were dried over Na₂SO₄ and filtered with suction.

After addition of 434.4 ml toluene the AcOEt was removed under reduced pressure such that precipitation of the product started. After removal of all AcOEt, the resulting suspension was cooled from 45°C to 5°C over a period of 45 min, the precipitate was filtered off with suction on a funnel with a fritted disk and washed with 65 ml toluene. The crystals were dried at 50°C *in vacuo* overnight to yield 18.98 g (85.9% of theory) of the title compound as light yellow crystals.

### Example 5.3: Synthesis of 5-Chloro-6-fluoro-1H-indole-7-carboxylic acid

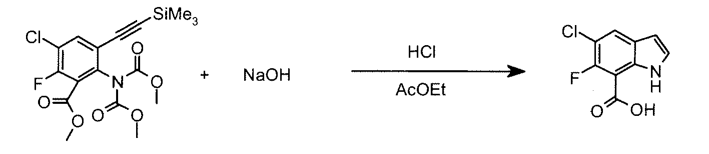

To a stirred solution of 5 g (12.02 mmol) 3-Chloro-2-fluoro-6-bis(methoxycarbonylamino)-5-trimethyl-silanylethynyl-benzoic acid methyl ester in 100 ml ethanol was added under argon 5.6 ml (60.10 mmol, 5 eq.) NaOH. The resulting thin suspension was heated at 80°C for 60 min, cooled to ambient temperature and the precipitate was filtered off with suction on a funnel with a fritted disk and washed with 5 ml ethanol. The wet filter cake was suspended in a mixture of 30 ml water and 20 ml AcOEt. Under vigorous stirring 15.0 ml (116.7 mmol, 25%, 9.71 eq.) HCl was added, such that the pH was adjusted to 1.

The organic phase was separated and washed with water, whereas the water phase was washed with AcOEt. The combined organic phases were dried over Na₂SO₄ and filtered with suction. After addition of 50 ml toluene the AcOEt was removed under reduced pressure such that precipitation of the product started. After removal of all AcOEt, the resulting suspension was stirred at ambient temperature for 0.5 h, the precipitate was filtered off with suction on a funnel with a fritted disk and washed with 5 ml toluene. The crystals were dried under at 50°C *in vacuo* to yield 7.78 g (69.3% of theory) of the title compound as white crystals.

### Example 5.4: Synthesis of 5-Chloro-6-fluoro-1H-indole-7-carboxylic acid

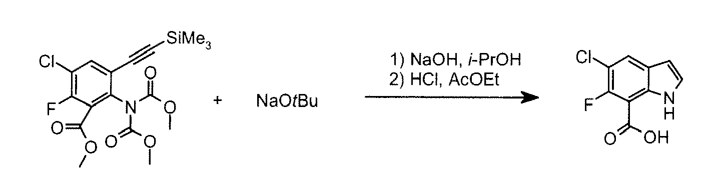

To a stirred solution of 10 g (24.04 mmol) 3-Chloro-2-fluoro-6-bis(methoxycarbonylamino)-5-trimethyl-silanylethynyl-benzoic acid methyl ester in 200 ml 2-propanol was added under argon 2.9 g (28.86 mmol, 1.2 eq.) NaOtBu. The resulting thin suspension was heated at 80°C for 2 h and 7.8 ml (84.14 mmol, 32%, 3.5 eq.) NaOH was added. The suspension was stirred at 80°C for 2 h, cooled to ambient temperature, and the precipitate was filtered with suction on a funnel with a fritted disk and washed with water. The wet filter cake was suspended in a mixture of 100 ml water and 80 ml AcOEt. Under vigorous stirring 30 ml (233.4 mmol, 25%, 9.71 eq.) HCl was added, such that the pH was adjusted to 1.

The organic phase was separated and washed with water, whereas the water phase was washed with AcOEt. The combined organic phases were dried over Na₂SO₄ and filtered with suction. After addition of 100 ml toluene the AcOEt was removed under redcued pressure such that precipitation of the product started. After removal of all AcOEt, the resulting suspension was stirred at ambient temperature for 0.5 h, the precipitate was filtered off with suction on a funnel with a fritted disk and washed with 20 ml toluene. The crystals were dried at 50°C *in vacuo* to yield 4.4 g (85.7% of theory) of the title compound as white crystals.

### Example 5.5: Synthesis of 5-Chloro-6-fluoro-1H-indole-7-carboxylic acid

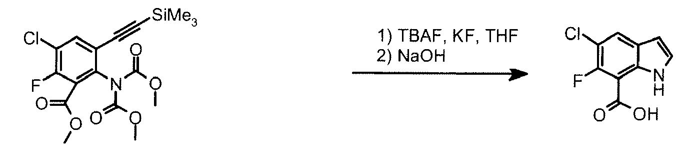

To a stirred solution of 1.0 g (2.3 mmol) 3-Chloro-2-fluoro-6-bis(methoxycarbonylamino)-5-trimethyl-silanylethynyl-benzoic acid methyl ester in 20 ml THF were added under argon 2.3 ml (2.3 mmol, 1.0 eq., 1M) TBAF and 135 mg (2.3 mmol, 1.0 eq.) potassium fluoride. The resulting black reaction mixture was stirred overnight at 42°C. To the reaction mixture was added 4.26 ml (46.00 mmol, 32%, 20 eq.) NaOH. The reaction mixture was stirred at 42°C overnight, and the solvent was removed to dryness and the resulting solid was dispersed in water/DCM. The organic layer was separated and acidified with HCl (37%), whereupon a suspension was formed. The precipitate was filtered with suction on a funnel with a fritted disk. The crystals were digested in chloroform and filtered again with suction on a funnel with a fritted disk and washed with ice cold chloroform. The crystals were dried at 50°C *in vacuo* to yield 0.4 g (81.5% of theory) of the title compound as light green crystals.

## Claims

1. A process for the preparation of an indole derivative of formula (I): 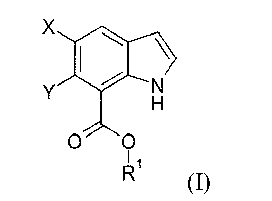 wherein
X is halogen, (C₁-C₆)alkoxy or (C₁-C₆)alkyl;
Y is halogen or (C₁-C₆)alkyl;
R¹ is (C₁-C₆)alkyl, (C₃-C₆)cycloalkyl, halo(C₁-C₆)alkyl, perhalo(C₁-C₆)alkyl, (C₁-C₆)alkenyl, (C₁-C₆)alkynyl, arylalkyl or optionally substituted aryl
which comprises the cyclization of formula (II) compound: 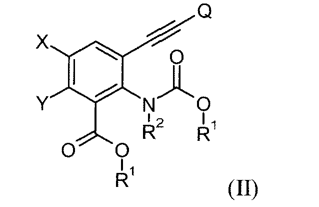 wherein X, Y and R¹ are as defined above, and
R² is halo(C₁-C₆)alkyl, perhalo(C₁-C₆)alkyl, -CO₂R, COR, or -SO₂R' wherein R and R¹ are (C₁-C₆)alkyl, phenyl(C₁-C₃)alkyl, (C₁-C₃)alkylphenyl orphenyl;
Q is -Si(R³)(R⁴)(R⁵) or -C(OH)(R⁴)(R⁵) wherein R³, R⁴ and R⁵ may be the same or different and are independently selected from (C₁-C₆)alkyl or phenyl;
with a cyclization reagent.

2. A process for the preparation of the compound of formula (II): 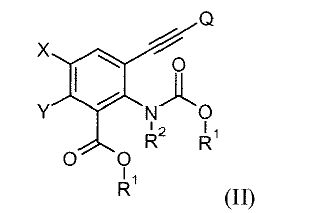 which comprises the carboxylation of the compound of formula (III): 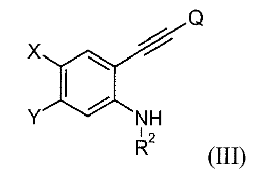 wherein X, Y, Q, R¹ and R² are as defined in claim 1, with a compound of formula Z-C(O)O- R¹ wherein Z is a halogen and R¹ is as defined in claim in the presence of alkalimetal-amine, optionally followed by the addition of an acid quenching reagent.

3. A process for the preparation of the compound of formula (IV), 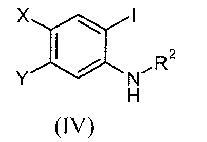 which comprises iodination of the compound of formula (V): 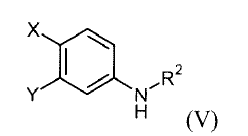 wherein X, Y and R² are as defined in claim 1, with an iodination source.

4. A process according to claim 1, which further comprises the preparation of the compound of formula (II): 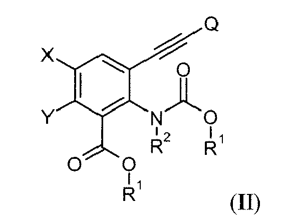 by the carboxylation of the compound of formula (III): 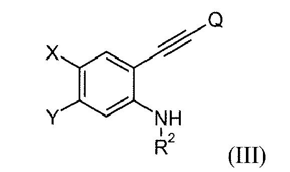 wherein X, Y, Q, R¹ and R² are as defined in claim 1, with a compound of formula Z-C(O)O- R¹ wherein R¹ is as defined in claim 1 in the presence of alkalimetal-amine, optionally followed by the addition of an acid quenching reagent.

5. A process according to either of claim 2 or 4, which further comprises the preparation of the compound of formula (III) by the reaction of Palladium catalyst and/or of copper catalyst in the presence of solvent with a compound of formula (IV) and acetylene of formula (B): 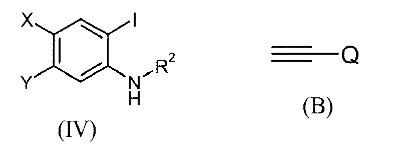 wherein X, Y, Q and R² are as defined in claim 1.

6. A process according to claim 5, which further comprises the preparation of the compound of formula (IV) by iodination of the compound of formula (V): 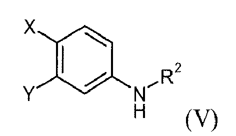 wherein X, Y and R² are as defined in claim 1, with an iodination source.

7. A process according to either claim 1, 2 or 4, wherein R¹ is (C₁-C₆)alkyl.

8. A process according to either claim 1 to 7, wherein R² is -COR and R is as defined in claim 1.

9. A process according to either claim 1, 2, 4, 7 or 8, wherein Q is -Si(R³)(R⁴)(R⁵) wherein R³,R⁴ and R⁵ are alkyl.

10. A process according to claim 1 wherein the cyclization reagent is alcoholate, alkalimetalhydroxyl, tetra(C₁-C₁₀)alkyl ammonium salts, alkali metal carbonates or a mixture thereof

11. A process according to either claim 2 or 4 wherein the quenching reagent is acetic acid, or citric acid.

12. A process according to claim 5 wherein the catalyst is a mixture of PdCl₂(PPh₃)₃ and CuI in the presence oftriethyl amine.

13. A process according to either claim 3, 6 or 9, wherein the iodination source is iodine/NaIO₄, iodine/H₅IO₆, N-iodo succinimide, *N*-iodo phthalimide, 1,3-diiodo-5,5-dimethylhydantoin, IC1 and HIO₃ or a mixture thereof.

14. A process for the preparation of an indole derivative of formula (I): 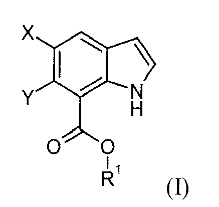 which comprises the cyclization of formula (II') compound: 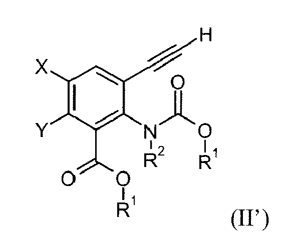 wherein X, Y, R¹ and R² are as defined in claim 1, with a cyclization reagent.

15. A compound of formula (II"): 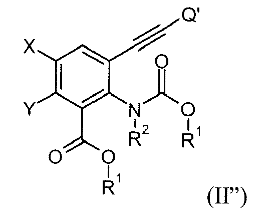 wherein Q' is H, -Si(R³)(R⁴)(R⁵) or -C(OH)(R⁴)(R⁵) and X, Y, R¹, R², R³, R⁴ and R⁵, are as defined in claim 1.

16. A compound of formula (II") according to claim 15, wherein X and Y are independently halogen or (C₁-C₃)alkyl.

17. A compound of formula (II") according to either claim 15 or 16, wherein R² is -CO₂R, and R' is (C₁-C₃)alkyl, where R is as defined in claim 1.

18. A compound of formula (II") according to either claim 16 or 17, wherein X is Cl and Y is F.
